(19) Europäisches Patentamt
European Patent Office
Office européen des brevets

(11) EP 4 220 187 A1

(12) EUROPEAN PATENT APPLICATION
published in accordance with Art. 153(4) EPC

(43) Date of publication:
02.08.2023 Bulletin 2023/31

(21) Application number: 21872558.8

(22) Date of filing: 24.09.2021

(51) International Patent Classification (IPC):
G01P 5/26 (2006.01)    G01F 1/66 (2022.01)
G01N 21/53 (2006.01)   A61B 5/0285 (2006.01)

(52) Cooperative Patent Classification (CPC):
A61B 5/0285; G01F 1/66; G01N 21/53; G01P 5/26

(86) International application number:
PCT/JP2021/035075

(87) International publication number:
WO 2022/065429 (31.03.2022 Gazette 2022/13)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.09.2020 JP 2020162391

(71) Applicant: Kyocera Corporation
Kyoto-shi Kyoto 612-8501 (JP)

(72) Inventors:
• ITOU Hiroshige
Kyoto-shi, Kyoto 612-8501 (JP)
• MATSUNAGA Shougo
Kyoto-shi, Kyoto 612-8501 (JP)

(74) Representative: Viering, Jentschura & Partner
mbB
Patent- und Rechtsanwälte
Am Brauhaus 8
01099 Dresden (DE)

(54) MEASURING DEVICE, MEASURING SYSTEM, MEASURING METHOD, AND PROGRAM

(57) A measurement device includes a light emitter, a light receiver, an identifier, and a calculator. The light emitter irradiates, with light, an irradiation target having a fluid flowing in an internal space of the irradiation target. The light receiver receives coherent light including light scattered by the irradiation target and outputs an output signal corresponding to an intensity of the coherent light. The identifier obtains a characteristic quantity indicating a characteristic of a signal waveform of the output signal, and identifies, based on the obtained characteristic quantity, an abnormal signal portion of the output signal. The calculator calculates, based on a signal portion of the output signal other than the abnormal signal portion, a flow state value indicating a flow state of the fluid.

FIG. 1

EP 4 220 187 A1

**Description**

CROSS-REFERENCE TO RELATED APPLICATION

This application claims priority to Japanese Patent Application No. 2020-162391 filed on September 28, 2020, the entire disclosure of which is incorporated herein by reference.

TECHNICAL FIELD

The present disclosure relates to a measurement technique.

BACKGROUND OF INVENTION

Techniques for detecting bubbles in blood are described in Japanese Unexamined Patent Application Publication Nos. 6-237998 and 2018-163163.

SUMMARY

One or more aspects of the disclosure are directed to a measurement device, a measurement system, a measurement method, and a program. In one embodiment, a measurement device includes a light emitter, a light receiver, an identifier, and a calculator. The light emitter irradiates, with light, an irradiation target having a fluid flowing in an internal space of the irradiation target. The light receiver receives coherent light including light scattered by the irradiation target and outputs an output signal corresponding to an intensity of the coherent light. The identifier obtains a characteristic quantity indicating a characteristic of a signal waveform of the output signal, and identifies, based on the obtained characteristic quantity, an abnormal signal portion of the output signal. The calculator calculates, based on a signal portion of the output signal other than the abnormal signal portion, a flow state value indicating a flow state of the fluid.

In one embodiment, a measurement system includes the measurement device described above, and a display device that displays the flow state value calculated by the measurement device.

In one embodiment, a measurement system includes the measurement device described above, and a display device. In response to the abnormal signal portion identified by the identifier, the measurement device outputs, to a device external to the measurement device, a notification signal indicating that the fluid contains foreign matter. The display device displays, in response to the notification signal output from the measurement device to the device external to the measurement device, notification information indicating that the fluid contains foreign matter.

In one embodiment, a measurement method is implementable with a device. The measurement method includes obtaining a characteristic quantity indicating a characteristic of a signal waveform of an output signal corresponding to an intensity of coherent light output from a light receiver receiving the coherent light, and identifying, based on the obtained characteristic quantity, an abnormal signal portion of the output signal. The coherent light includes light scattered by an irradiation target irradiated with light by a light emitter. The irradiation target has a fluid flowing in an internal space of the irradiation target. The measurement method further includes calculating, based on a signal portion of the output signal other than the abnormal signal portion, a flow state value indicating a flow state of the fluid.

In one embodiment, a program is executable by a computer to receive an input of an output signal corresponding to an intensity of coherent light output from a light receiver receiving the coherent light. The coherent light includes light scattered by an irradiation target irradiated with light by a light emitter. The irradiation target has a fluid flowing in an internal space of the irradiation target. The program causes the computer to perform operations including obtaining a characteristic quantity indicating a characteristic of a signal waveform of the output signal, and identifying, based on the obtained characteristic quantity, an abnormal signal portion of the output signal, and calculating, based on a signal portion of the output signal other than the abnormal signal portion, a flow state value indicating a flow state of the fluid.

BRIEF DESCRIPTION OF THE DRAWINGS

FIG. 1 is a schematic diagram of an example measurement device.
FIG. 2 is a schematic partial block diagram of the measurement device.
FIG. 3 is a schematic block diagram of an example arithmetic circuit.
FIG. 4 is a flowchart of an example operation performed by the arithmetic circuit.
FIG. 5 is a schematic graph showing an example power spectrum.
FIG. 6 is a graph showing an example signal waveform of an AC signal.
FIG. 7 is a graph showing an example signal waveform of the AC signal.
FIG. 8 is a graph showing an example signal waveform of the AC signal.
FIG. 9 is a graph showing an example signal waveform of the AC signal.
FIG. 10 is a graph showing an example signal waveform of the AC signal.
FIG. 11 is a graph showing an example signal waveform of the AC signal.
FIG. 12 is a graph showing an example signal waveform of the AC signal.
FIG. 13 is a graph showing an example signal waveform of the AC signal.
FIG. 14 is a table showing example characteristic quantities of the signal waveform of the AC signal.
FIG. 15 is a table showing example characteristic quantities of the signal waveform of the AC signal.
FIG. 16 is a table showing example characteristic quantities of the signal waveform of the AC signal.
FIG. 17 is a table showing example characteristic quantities of the signal waveform of the AC signal.
FIG. 18 is a table showing example characteristic quantities of the signal waveform of the AC signal.
FIG. 19 is a table showing example characteristic quantities of the signal waveform of the AC signal.
FIG. 20 is a table showing example characteristic quantities of the signal waveform of the AC signal.
FIG. 21 is a table showing example characteristic quantities of the signal waveform of the AC signal.
FIG. 22 is a table showing example characteristic quantities of the signal waveform of the AC signal.
FIG. 23 is a table showing example characteristic quantities of the signal waveform of the AC signal.
FIG. 24 is a table showing example characteristic quantities of the signal waveform of the AC signal.
FIG. 25 is a table showing example characteristic quantities of the signal waveform of the AC signal.
FIG. 26 is a table showing example characteristic quantities of the signal waveform of the AC signal.
FIG. 27 is a table showing example characteristic quantities of the signal waveform of the AC signal.
FIG. 28 is a table showing example characteristic quantities of the signal waveform of the AC signal.
FIG. 29 is a table showing example characteristic quantities of the signal waveform of the AC signal.
FIG. 30 is a table showing example characteristic quantities of the signal waveform of the AC signal.
FIG. 31 is a table showing example characteristic quantities of the signal waveform of the AC signal.
FIG. 32 is a table showing example characteristic quantities of the signal waveform of the AC signal.
FIG. 33 is a table showing example characteristic quantities of the signal waveform of the AC signal.
FIG. 34 is a table showing example characteristic quantities of the signal waveform of the AC signal.
FIG. 35 is a table showing example characteristic quantities of the signal waveform of the AC signal.
FIG. 36 is a table showing example characteristic quantities of the signal waveform of the AC signal.
FIG. 37 is a table showing example characteristic quantities of the signal waveform of the AC signal.
FIG. 38 is a schematic diagram of an example measurement system.

DESCRIPTION OF EMBODIMENTS

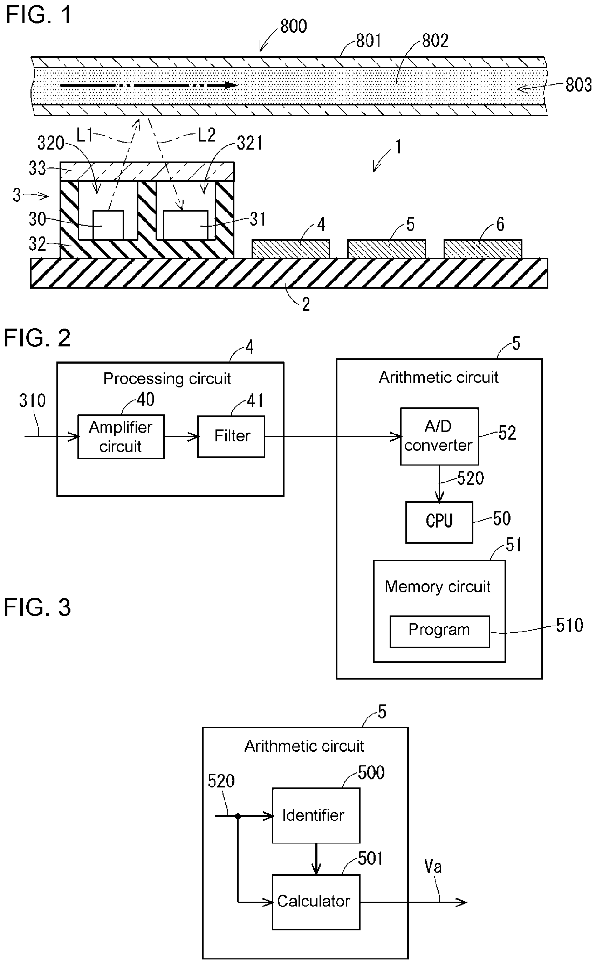

FIG. 1 is a schematic diagram of an example measurement device 1. As illustrated in FIG. 1, for example, the measurement device 1 can irradiate, with light L1, an object (also referred to as an irradiation target) 800 having a fluid 802 flowing in an internal space 803. The irradiation target 800 includes an object (flow path component) 801 defining a flow path, and the fluid 802 flowing through the flow path. The internal space 803 of the flow path component 801 is the internal space 803 of the irradiation target 800. The measurement device 1 can receive coherent light including light scattered by the irradiation target 800 and quantitatively measure the flow state of the fluid 802 based on the received coherent light. In other words, the measurement device 1 can calculate a quantitative value (also referred to as a flow state value) indicating the flow state of the fluid 802. The flow path component 801 includes, for example, a tubular object (also referred to as a tubular body) such as a blood vessel in a living body or a pipe in any of various devices. The flow state value includes, for example, at least one of the flow rate or the flow velocity. The flow rate is, for example, the quantity of a fluid that passes through the flow path per unit time. The quantity of the fluid is expressed in, for example, volume or mass. The flow velocity is the velocity of the fluid flowing through the flow path. The flow velocity is expressed with, for example, a distance by which the fluid flows per unit time.

The measurement device 1 can quantitatively measure the flow state of the fluid 802 with, for example, the Doppler effect for light. When, for example, light incident on the fluid 802 is scattered by the fluid 802, the Doppler effect corresponding to the flow of the fluid 802 causes a frequency shift (also referred to as a Doppler shift) of the light corresponding to the movement speed of the fluid 802. The measurement device 1 can quantitatively measure the flow state of the fluid 802 with this Doppler shift.

The fluid 802 as a target (also referred to as a measurement target) for which the flow state is measured quantitatively may be, for example, a fluid 802 that scatters light. In some embodiments, the fluid 802 may be a fluid that allows a substance or an object that scatters light (also referred to as a scatter substance or a scatterer) to flow through the fluid. Examples of the fluid 802 as a measurement target include water, blood, printer ink, various water solutions such as beverages, and gas containing a scatterer such as powder. For a scatter substance or a scatterer flowing with the fluid, the flow rate of the scatter substance or the scatterer may be used as the flow rate of the fluid, and the flow velocity of the scatter substance or the scatterer may be used as the flow velocity of the fluid.

As illustrated in FIG. 1, the measurement device 1 includes, for example, a wiring board 2, a sensor 3, a processing circuit 4, an arithmetic circuit 5, and a connector 6. The measurement device 1 may include an outer case accommodating the wiring board 2, the sensor 3, the processing circuit 4, the arithmetic circuit 5, and the connector 6.

The wiring board 2 includes, for example, the sensor 3, the processing circuit 4, the arithmetic circuit 5, and the connector 6 on its first surface. The wiring board 2 may be a monolayer board or a multilayer board. At least one of the processing circuit 4, the arithmetic circuit 5, or the connector 6 may be located on a second surface of the wiring board 2 opposite to the first surface.

For example, the connector 6 is connected to a cable extending from a device external to the measurement device 1. The connector 6 receives, for example, power supplied from a power supply for the measurement device 1. The power supplied to the connector 6 is then supplied to the sensor 3, the processing circuit 4, and the arithmetic circuit 5 through the wiring board 2.

The sensor 3 includes, for example, a light emitter 30 and a light receiver 31. The light emitter 30 can irradiate the irradiation target 800 with the light L1. The light L1 from the light emitter 30 to be incident on the irradiation target 800 may be, for example, light having a predetermined wavelength as appropriate for the fluid 802. For the fluid 802 being blood, for example, the light L1 having a wavelength set to about 600 to 900 nanometers (nm) is used. For the fluid 802 being printer ink, for example, the light L1 having a wavelength set to about 700 to 1000 nm is used. The light emitter 30 may be, for example, a semiconductor laser device, such as a vertical-cavity surface-emitting laser (VCSEL).

The light receiver 31 can receive, for example, coherent light L2 including a portion of light L1 scattered by the irradiation target 800 after being emitted from the light emitter 30. The light receiver 31 can convert, for example, the received light to an electric signal corresponding to the light intensity. In other words, the light receiver 31 can receive coherent light L2 including light scattered by the irradiation target 800 and output a signal in the time domain corresponding to the intensity of the coherent light L2. Of the scattered light from the irradiation target 800, coherent light L2 that can be received by the light receiver 31 includes, for example, scattered light without a Doppler shift from an object that is stationary around the fluid 802 (also referred to as a stationary object) and scattered light with a Doppler shift Δf from the fluid 802. For the fluid 802 being blood flowing through a blood vessel, for example, the stationary object includes the skin and the blood vessel. For the fluid 802 being ink flowing through a pipe, the stationary object includes the pipe. In this case, the pipe is made of, for example, a light-transmissive material. Examples of the light-transmissive material include glass and a polymer resin.

A change in the intensity of the coherent light L2 with time (also referred to as a temporal change) indicates a beat of the frequency corresponding to a difference (also referred to as a difference frequency) Δf between the frequency of the scattered light without a Doppler shift and the frequency of the scattered light with a Doppler shift. Thus, the output signal corresponding to the intensity of the coherent light L2 output from the light receiver 31 can contain a component of a signal corresponding to the beat (also referred to as a beat signal or an optical beat signal) with respect to the temporal change in the intensity of the coherent light L2. The light receiver 31 may be, for example, any device that can follow the beat (also referred to as having time resolution) with respect to the temporal change in the intensity of the coherent light L2. The wavelength of light that can be received by the light receiver 31 can be set based on measurement conditions such as the wavelength of light L1 emitted from the light emitter 30 to be incident on the irradiation target 800 and the velocity range of the fluid 802. The light receiver 31 may be, for example, a silicon (Si) photodiode, a gallium arsenide (GaAs) photodiode, an indium gallium arsenide (InGaAs) photodiode, or a germanium (Ge) photodiode.

The sensor 3 includes, for example, a package 32 and a cover 33, in addition to the light emitter 30 and the light receiver 31. The package 32 accommodates the light emitter 30 and the light receiver 31. The package 32 includes a recess 320 accommodating the light emitter 30 and a recess 321 accommodating the light receiver 31. The cover 33 covers the recesses 320 and 321 in the package 32.

The package 32 may be, for example, a multilayered wiring board made of a ceramic material or an organic material. The multilayered wiring board electrically connects the light emitter 30 and the light receiver 31 to the wiring board 2. Examples of the ceramic material include sintered aluminum oxide and sintered mullite. Examples of the organic material include an epoxy resin and a polyimide resin.

The cover 33 is, for example, a transparent glass plate. Light L1 emitted from the light emitter 30 in the recess 320 is incident on the irradiation target 800 through the cover 33. Coherent light L2 from the irradiation target 800 is received by the light receiver 31 in the recess 321 through the cover 33.

The processing circuit 4 is electrically connected to the light receiver 31 with the multilayer wiring board included in the package 32 for the sensor 3 and with the wiring board 2. The arithmetic circuit 5 is electrically connected to the processing circuit 4 with the wiring board 2.

FIG. 2 is a schematic block diagram of the processing circuit 4 and the arithmetic circuit 5. As illustrated in FIG. 2, the processing circuit 4 includes, for example, an amplifier circuit 40 and a filter 41. The amplifier circuit 40 amplifies and outputs an output signal 310 from the light receiver 31. The amplifier circuit 40 includes, for example, an operational amplifier, a resistor, and a capacitor. The filter 41 performs filtering of the output signal 310 amplified by the amplifier circuit 40. The output signal 310 filtered by the filter 41 is input into the arithmetic circuit 5. The filter 41 includes, for example, an operational amplifier, a resistor, and a capacitor.

The filter 41 is, for example, a high-pass filter that removes a direct current (DC) component from the amplified output signal 310 and outputs the resultant signal. In other words, the filter 41 extracts an alternating current (AC) component from the output signal 310 and outputs the resultant signal. The output signal 310, or specifically the AC component in the output signal 310, filtered by and output from the filter 41 may also be referred to as an AC signal.

The processing circuit 4 may control power supply to the light emitter 30 to control emission from the light emitter 30. The processing circuit 4 may also include an amplifier circuit that amplifies the AC signal output from filter 41 and outputs the resultant signal to the arithmetic circuit 5.

The arithmetic circuit 5 includes, for example, at least one processor. In various embodiments, the processor(s) may be a single integrated circuit (IC), multiple ICs connected to one another for mutual communication, or discrete circuits. The processor(s) can be implemented in accordance with various known techniques.

In one embodiment, for example, the processor includes one or more circuits or units that execute instructions stored in an associated memory to perform one or more data computation procedures or processes. In another embodiment, the processor may be firmware (e.g., a discrete logic component) that performs one or more data computation procedures or processes.

In various embodiments, the processor includes one or more processors, controllers, microprocessors, microcontrollers, application-specific integrated circuits (ASICs), digital signal processors, programmable logic devices, field programmable gate arrays, combinations of any of these devices or configurations, or combinations of other known devices and configurations, and may implement the functions described below.

In the present example, the arithmetic circuit 5 includes, for example, a microprocessor. The microprocessor is a computer and includes, for example, a central processing unit (CPU) 50, a memory circuit 51, and an A/D converter 52, as illustrated in FIG. 2. The CPU 50 is also a computer. The memory circuit 51 includes, for example, a non-transitory recording medium readable by the CPU 50, such as a read-only memory (ROM) and a random-access memory (RAM). The memory circuit 51 stores a program 510. The CPU 50 executing the program 510 in the memory circuit 51 implements the various functions of the arithmetic circuit 5. The computer included in the arithmetic circuit 5 may include at least one of the functions of the processing circuit 4.

The program 510 is stored in a non-transitory computer-readable storage medium included in the memory circuit 51. The non-transitory computer-readable storage medium storing the program 510 may be a portable storage medium such as an optical disk, a magnetic disk, or a nonvolatile memory. In this case, for example, the portable storage medium may be attached to a device, such as a disk drive or a memory reader, that can read data. The program 510 may be stored into the portable storage medium.

The A/D converter 52 can convert an analog AC signal input from the processing circuit 4 to a digital AC signal. The CPU 50 can quantitatively measure the flow state of the fluid 802 based on a digital AC signal (also referred to as an AC signal 520). The CPU 50 calculates the flow state value based on the AC signal 520.

The AC signal 520 is a time-domain signal and includes multiple time-series digital values indicating the signal strength of the AC signal 520. The A/D converter 52 has a resolution of, for example, 12 bits, and outputs digital values in the range of 0 to 4095 in decimal notation. The A/D converter 52 outputs digital values at sampling intervals. The multiple digital values included in the AC signal 520 are at sampling intervals of the A/D converter 52. The A/D converter 52 may have a resolution other than 12 bits. The digital values included in the AC signal 520 may be hereafter referred to as AC values. Each AC value indicates the signal strength of the AC signal 520.

FIG. 3 is a block diagram of the arithmetic circuit 5, illustrating example functional blocks implemented by the arithmetic circuit 5 when the CPU 50 executes the program 510. As illustrated in FIG. 3, the arithmetic circuit 5 includes, for example, an identifier 500 and a calculator 501 as functional blocks. The identifier 500 and the calculator 501 are implemented by the CPU 50.

The fluid 802 can contain foreign matter. For the fluid 802 being blood, for example, the fluid 802 may contain bubbles as foreign matter. When the light receiver 31 receives light scattered by foreign matter in the fluid 802, the AC signal 520 may contain an abnormal signal portion. The flow state value calculated using the AC signal 520 containing an abnormal signal portion can have lower accuracy, thus lowering the measurement accuracy of the flow state of the fluid 802.

The identifier 500 identifies an abnormal signal portion of the AC signal 520. The identifier 500 obtains, for example, a characteristic quantity indicating the characteristics of the signal waveform of the AC signal 520. The identifier 500 identifies an abnormal signal portion of the AC signal 520 based on the obtained characteristic quantity. The calculator 501 calculates and outputs a flow state value Va based on signal portions of the AC signal 520 other than the abnormal signal portion identified by the identifier 500. The measurement method includes the process of identifying an abnormal signal portion and the process of calculating the flow state value Va described above. The program 510 causes the computer to perform the process of identifying an abnormal signal portion and calculating the flow state value Va described above.

Thus, the signal portions other than the abnormal signal portion of the AC signal 520 are used to calculate the flow state value Va to increase the calculation accuracy. This increases the measurement accuracy of the flow state of the fluid 802. Example operations performed by the identifier 500 and the calculator 501 will now be described in detail.

For example, the identifier 500 periodically calculates, for each unit period T, the characteristic quantity of the signal waveform (also referred to as a first signal waveform) of a signal portion (also referred to as a first signal portion) of the AC signal 520. The identifier 500 then periodically determines, for each unit period T, whether the first signal portion is an abnormal signal portion based on the characteristic quantity calculated for the unit period T. When the identifier 500 determines that the first signal portion is not an abnormal signal portion for a unit period T, for example, the calculator 501 uses this first signal portion to calculate the flow state value Va. In other words, when the identifier 500 determines that the first signal portion is normal for any unit period T, the calculator 501 uses this first signal portion to calculate the flow state value Va. When the identifier 500 determines that the first signal portion is an abnormal signal portion for any unit period T, the calculator 501 does not use this first signal portion to calculate the flow state value Va. The unit period T being in focus may be hereafter referred to as a target unit period T.

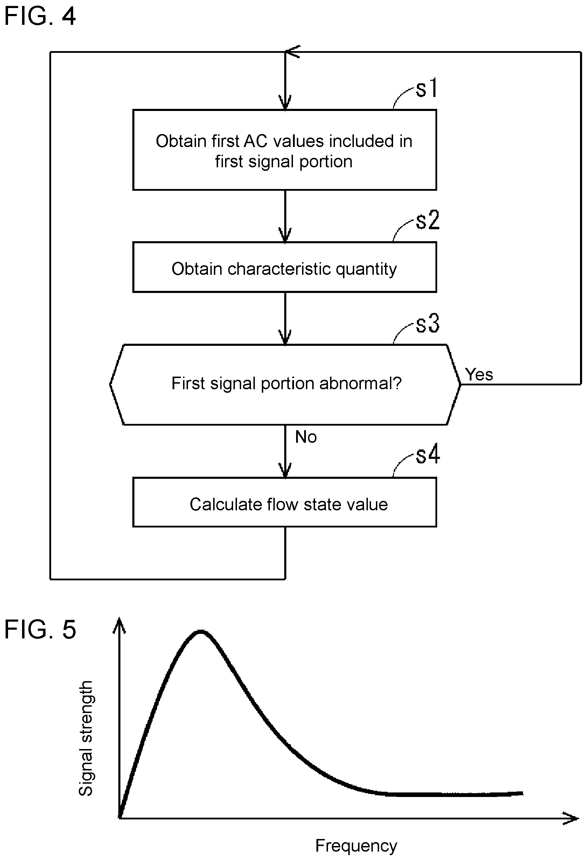

FIG. 4 is a flowchart of an example operation performed by the arithmetic circuit 5 for calculating the flow state value Va. The processing in steps s1 to s4 shown in FIG. 4 is performed periodically for each unit period T.

In the present example, adjacent unit periods T are not continuous with each other but have a time difference between the end of the preceding unit period T and the start of the following unit period T. The unit period T is set to, for example, a period for which N AC values are output from the A/D converter 52 (N is an integer greater than or equal to 2). The first signal portion includes time-series N AC values. The arithmetic circuit 5 obtains N AC values for each unit period T.

For N being 1024, for example, the unit period T is set to the period for which 1024 AC values are output from the A/D converter 52. In this case, the A/D converter 52 may have a sampling frequency of 100 kHz and a sampling interval of 10 $\mu$s. The unit period T is thus about 10.2 ms. The first signal portion for the unit period T includes time-series 1024 AC values. The sampling frequency of the A/D converter 52 is hereafter simply referred to as a sampling frequency.

As illustrated in FIG. 4, in step s1, the identifier 500 obtains N AC values included in the first signal portion for the target unit period T. The identifier 500 identifies, for example, the latest N AC values output from the A/D converter 52 as N AC values included in the first signal portion for the target unit period T. The start and the end of step s1 are the start and the end of the target unit period T.

In step s2, the identifier 500 obtains the signal waveform of the first signal portion for the target unit period T based on the N AC values obtained in step s 1. In other words, the identifier 500 obtains the characteristic quantity indicating the characteristics of the first signal waveform for the target unit period T. In step s3, the identifier 500 determines, based on the characteristic quantity obtained in step s2, whether the first signal portion for the target unit period T is an abnormal signal portion. The processing in steps s2 and s3 may be hereafter collectively referred to as abnormality determination. The abnormality determination will be described in detail later.

In response to a negative determination result in step s3, the calculator 501 calculates, in step s4, the flow state value Va based on the first signal portion for the target unit period T. More specifically, the calculator 501 calculates the flow state value Va based on the N AC values included in the first signal portion obtained in step s1. A specific example of step s4 will now be described. A first signal portion determined not to be an abnormal signal portion includes AC values that may be hereafter referred to as normal AC values.

The frequency and the signal strength of the output signal 310 from the light receiver 31 vary depending on the Doppler effect for light. Thus, the frequency spectrum showing the relationship between the frequency and the signal strength of the output signal 310 changes in accordance with the flow state value Va (e.g., the flow rate or the flow velocity) of the fluid 802. Thus, in step s4, the calculator 501 performs a Fourier transform on the first signal portion obtained from the output signal 310 to calculate the power spectrum of the first signal portion. More specifically, the calculator 501 performs a discrete Fourier transform on N normal AC values included in the first signal portion that are determined not to be an abnormal signal portion. The calculator 501 thus calculates the power spectrum of the first signal portion.

FIG. 5 is a graph showing an example power spectrum calculated by the calculator 501. In FIG. 5, the horizontal axis represents the frequency, and the vertical axis represents the signal strength of the first signal portion at each frequency.

The calculator 501 calculates the flow state value Va based on the calculated power spectrum. In this calculation, fn represents frequency, and P(fn) represents the signal strength of the first signal portion at the frequency fn. For example, the calculator 501 integrates the signal strength P(fn) that is weighted using the frequency fn to calculate a calculation value Vb. The calculation value Vb is written in Formula 1 below. The calculation value Vb changes in accordance with the flow state of the fluid 802.

$$Vb = \sum fn \times P(fn) \qquad (1)$$

The calculator 501 calculates a quantitative value indicating the flow state of the fluid 802, or specifically the flow state value Va, based on the calculated calculation value Vb. For example, the calculator 501 calculates the flow state value Va based on the calculated calculation value Vb and based on prepared calibration data (also referred to as a calibration curve). For example, the calibration data is prestored in the memory circuit 51 in the arithmetic circuit 5 before the flow state value Va of the fluid 802 is calculated. The calibration data may be stored in the form of, for example, a functional formula or a table. With calibration data about the flow rate of the fluid 802 being stored in the memory circuit 51, the calculator 501 can calculate the flow rate of the fluid 802 based on the calculation value Vb and based on the calibration data stored in the memory circuit 51. With calibration data about the flow velocity of the fluid 802 being stored in the memory circuit 51, the calculator 501 can calculate the flow velocity of the fluid 802 based on the calculation value Vb and based on the calibration data stored in the memory circuit 51.

Following step s4, step s1 is performed again. In step s1, the identifier 500 obtains N AC values included in the first signal portion for the subsequent unit period T. The identifier 500 identifies the latest N AC values output from the A/D converter 52 as N AC values included in the first signal portion for the subsequent unit period T. The arithmetic circuit 5 then performs the processing in step s2 and thereafter operates in the same manner as described above.

In response to an affirmative determination result in step s3, step s1 is performed again without the flow state value Va being calculated. In step s1, the identifier 500 obtains N AC values included in the first signal portion for the subsequent unit period T, similarly to the processing in step s1 following step s4. The arithmetic circuit 5 then performs the processing in step s2 and thereafter operates in the same manner as described above.

The arithmetic circuit 5 may repeat the process shown in FIG. 4 constantly or during a predetermined period while the measurement device 1 is active.

As described above, in the present example, the start and the end of step s1 for the target unit period T are the start and the end of the target unit period T. For step s4 being performed for the target unit period T, for example, the start and the end of step s1 performed immediately after step s4 are the start and the end of the unit period T subsequent to the target unit period T. In response to an affirmative determination result in step s3 for the target unit period T, the start and the end of step s1 performed immediately after step s3 are the start and the end of the unit period T subsequent to the target unit period T.

The delay time between the end of the target unit period T and the start of the subsequent unit period T (also referred to as a delay time for the unit period T) is substantially equal to the time taken to perform the processing in steps s2, s3, and s4 or the processing in steps s2 and s3. For step s4 being performed for the target unit period T, for example, the delay time from the end of the target unit period T to the start of the subsequent unit period T is substantially equal to the time taken to perform the processing in steps s2 to s4 for the target unit period T. In response to an affirmative determination result in step s3 for the target unit period T, the delay time from the end of the target unit period T to the start of the subsequent unit period T is substantially equal to the time taken to perform the processing in steps s2 and s3 for the target unit period T. The delay time for the unit period T varies depending on the processing speed of the CPU 50. The delay time for the unit period T is, for example, sufficiently smaller than the length of the unit period T. The delay time for the unit period T is, for example, tens to hundreds of microseconds.

The calculator 501 may calculate the flow state value Va in a manner other than the above manner. For example, the calculator 501 may use the calculation value Vb written in Formula 1 as the flow state value Va. The calculation value Vb can also indicate the flow state of the fluid 802. In this case, a device external to the measurement device 1 may calculate the flow rate or the flow velocity of the fluid 802 based on the calculation value Vb calculated by the calculator 501. The calculation value Vb as the flow state value Va calculated by the calculator 501 may be provided to the external device through the connector 6.

Examples of the abnormality determination (steps s2 and s3) will now be described. The first signal portion for the target unit period T may be hereafter referred to as a target first signal portion. The signal waveform of the target first signal portion may be hereafter referred to as a target first signal waveform. In the abnormality determination for the target unit period T, the determination is performed as to whether the target first signal portion is an abnormal signal portion. The unit period T for which the first signal portion is determined to be an abnormal signal portion may be hereafter referred to as an abnormal unit period T. The unit period T for which the first signal portion is determined not to be an abnormal signal portion may be hereafter referred to as a normal unit period T.

First Example

In the abnormality determination in the present example, the identifier 500 calculates a standard deviation σu corresponding to the target first signal waveform in step s2 for the target unit period T. The standard deviation σu may be the standard deviation for the N AC values included in the target first signal portion. The identifier 500 uses the calculated standard deviation σu as the characteristic quantity of the target first signal waveform.

In step s3 for the target unit period T, the identifier 500 performs a process of determining whether the target first signal portion is an abnormal signal portion (also referred to as first determination) based on the standard deviation σu. In the first determination, for example, the identifier 500 calculates an average σuave of the standard deviations σu calculated for M unit periods T preceding the target unit period T. M is an integer greater than or equal to 2. The M target unit periods T for which the average σuave is calculated in step s3 for the target unit period T may be hereafter referred to as M target unit periods T.

The M target unit periods T may be, for example, M normal unit periods T closest to the target unit period T. For example, the unit periods T1 to T8 may occur in sequence in this order, and M = 5. Of the unit periods T1 to T8, the unit period T4 may be an abnormal unit period T. In this case, the abnormality determination for the target unit period T8 determines that the five normal unit periods T closest to the target unit period T8, or specifically, the unit periods T7, T6, T5, T3, and T2, are the M target unit periods T.

In the first determination, the identifier 500 calculates an average σuave and compares the average σuave with the standard deviation σu. The identifier 500 uses the comparison result to determine whether the target first signal portion is an abnormal signal portion. For example, the identifier 500 calculates a ratio MG of the standard deviation σu to the average σuave. The ratio MG is expressed by σu/σuave. When the ratio MG is greater than or equal to a first threshold, the identifier 500 determines that the target first signal portion is an abnormal signal portion. When the ratio MG is less than the first threshold, the identifier 500 determines that the target first signal portion is not an abnormal signal portion. The identifier 500 may determine that the target first signal portion is an abnormal signal portion when the ratio MG is greater than the first threshold, and may determine that the target first signal portion is not an abnormal signal portion when the ratio MG is less than or equal to the first threshold.

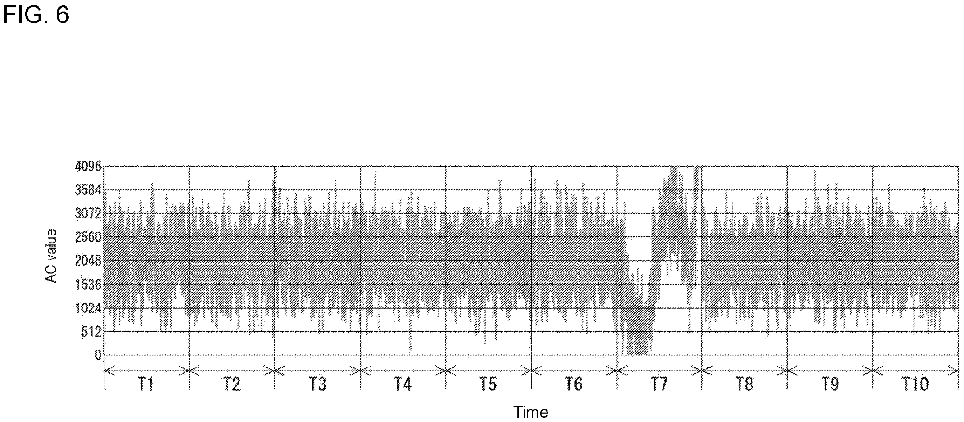

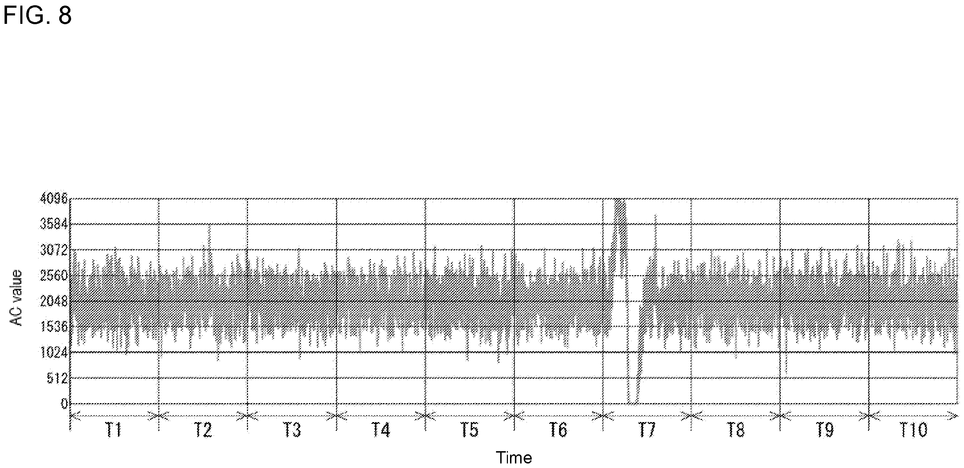

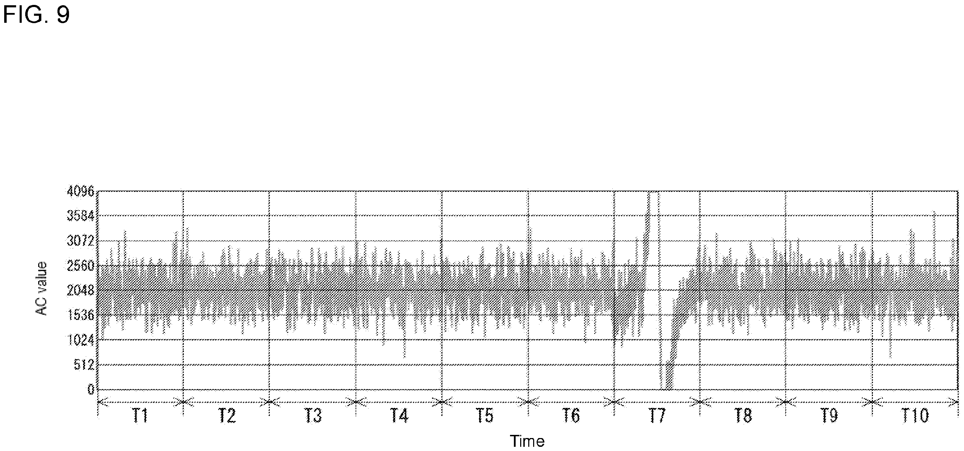

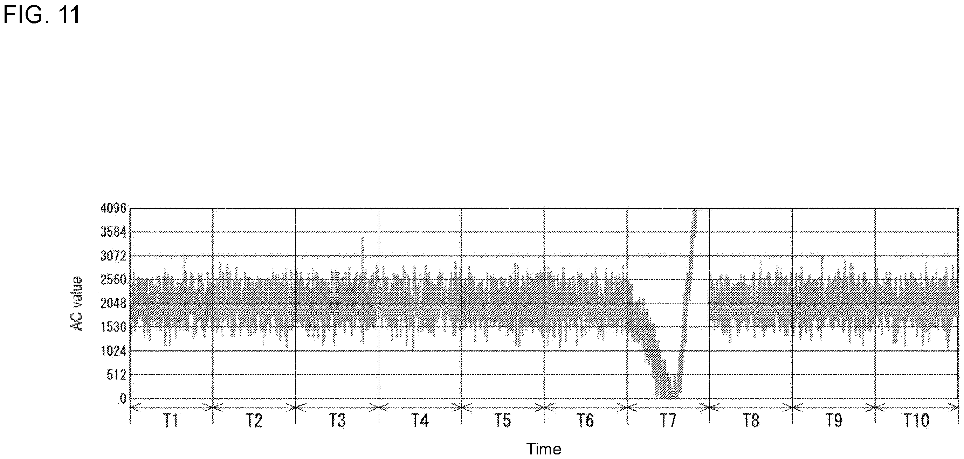

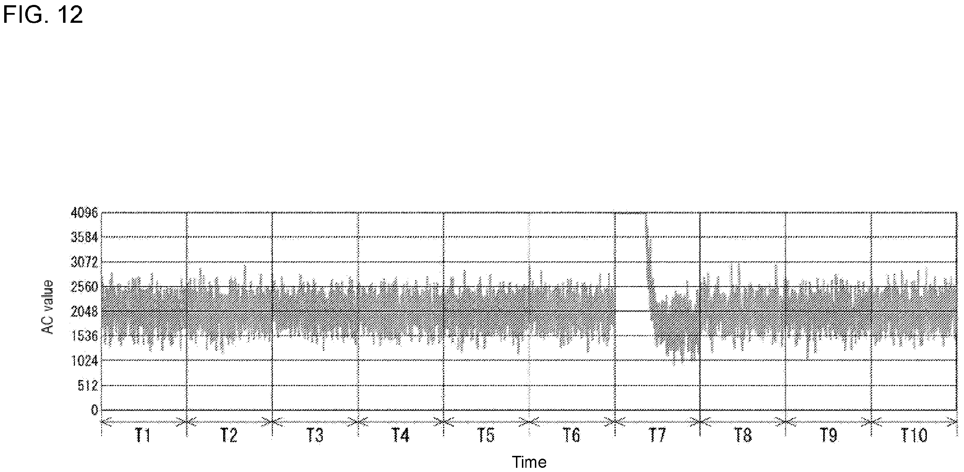

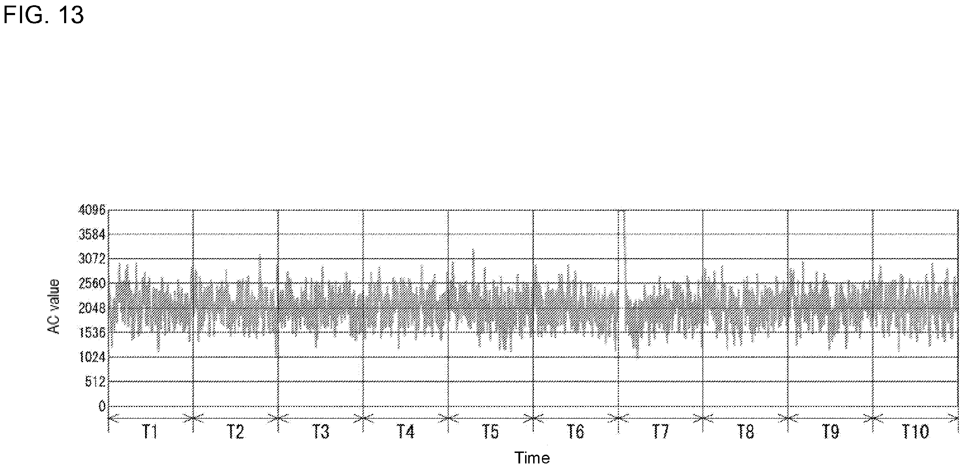

FIGs. 6 to 13 are graphs each showing an example signal waveform of the AC signal 520 when the light scattered by foreign matter in the fluid 802 is received by the light receiver 31. In FIGs. 6 to 13, the horizontal axis represents time, and the vertical axis represents the AC value (or in other words, the signal strength). FIGs. 6 to 13 each show the first signal waveforms for the unit periods T1 to T10. The unit periods T1 to T10 occur in sequence in this order in the measurement device 1. In the measurement device 1, the first signal waveforms for the unit periods T1 to T10 are not continuous with one another due to a delay time for each unit period T. In FIGs. 6 to 13, the first signal waveforms for the unit periods T1 to T10 are continuous with one another for ease of explanation.

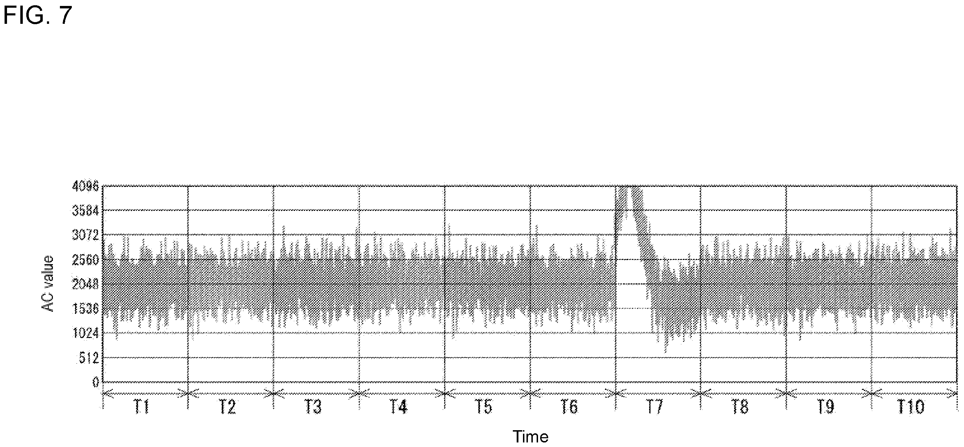

FIGs. 6 to 13 each show the signal waveform for the flow path component 801 being a perfluoroalkoxy alkane (PFA) tube and the fluid 802 being simulated blood. The simulated blood as the fluid 802 is an aqueous solution containing numerous spherical polymers with a diameter of 10 μm and a polymer content of about 45 vol%. FIG. 6 shows the signal waveform for the PFA tube as the flow path component 801 with an outer diameter of 4 mm and an inner diameter of 3 mm. FIGs. 7 to 13 each show the signal waveform for the PFA tube as the flow path component 801 with an outer diameter of 4 mm and an inner diameter of 2 mm.

FIG. 6 shows the signal waveform for the fluid 802 containing, as foreign matter, a single air bubble being substantially spherical and having a diameter of about 2 mm. FIG. 6 shows the signal waveform for the light emitter 30 with a light quantity of 2.0 mW, a sampling frequency of 50 kHz, and the fluid 802 flowing at a flow rate of 100 ml/min. FIG. 6 shows the signal waveform for N = 1024 with the unit period T being about 20.5 ms. In the example of FIG. 6, the bubble in the fluid 802 takes about 21.2 ms to pass through the light-receiving area in the light receiver 31.

FIGs. 7 to 13 each show the signal waveform for the fluid 802 containing, as foreign matter, a single black plastic piece with a diameter of 0.8 mm. FIGs. 7 to 13 each show the signal waveform for the light emitter 30 with a light quantity of 1.5 mW.

FIGs. 7 to 9 each show the signal waveform for the fluid 802 flowing at a flow rate of 20 ml/min. FIGs. 7 to 9 show the signal waveforms for the respective sampling frequencies of 50, 100, and 200 kHz. FIGs. 7 to 9 show the signal waveforms for N = 1024 with the respective unit periods T being about 20.5 ms, about 10.2 ms, and about 5.1 ms. In the examples of FIGs. 7 to 9, the black plastic piece in the fluid 802 takes about 35.8 ms to pass through the light-receiving area in the light receiver 31.

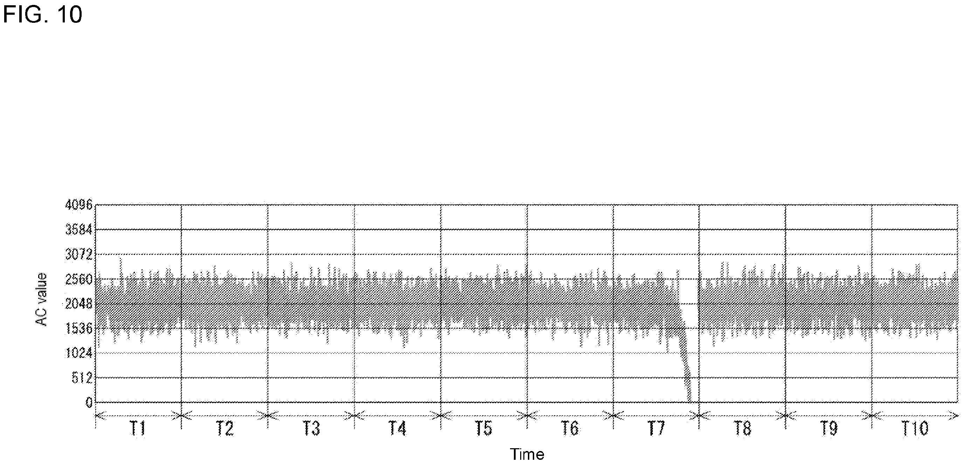

FIGs. 10 to 13 each show the signal waveform for the fluid 802 with a flow velocity of 50 ml/min. FIGs. 10 to 13 show the signal waveforms for the respective sampling frequencies of 50, 100, 200, and 400 kHz. FIGs. 10 to 13 show the signal waveforms for N = 1024 with the respective unit periods T being about 20.5 ms, about 10.2 ms, about 5.1 ms, and about 2.6 ms. In the examples of FIGs. 10 to 13, the black plastic piece in the fluid 802 takes about 14.3 ms to pass through the light-receiving area in the light receiver 31.

In the examples of FIGs. 6 to 13, the light receiver 31 receives scattered light from the foreign matter for the unit period T7 and receives no such light for the unit periods T1 to T6 and T8 to T10. Scattered light from foreign matter thus affects the first signal waveform for the unit period T7 without affecting the first signal waveforms for the unit periods T1 to T6 and T8 to T10. The first signal portion for the unit period T7 is an abnormal signal portion.

The fluid 802 has a non-uniform flow velocity that varies depending on the location in the flow path component 801. The fluid 802 tends to have a lower flow velocity when being nearer the inner wall of the flow path component 801. The light receiver 31 thus receives coherent light L2 including multiple rays of scattered light with different Doppler shifts. In other words, the light receiver 31 receives coherent light L2 including multiple rays of scattered light with different frequencies. The AC signal 520 is thus a combination of multiple AC signal components with different frequencies.

The AC signal 520 unaffected by scattered light from foreign matter has a waveform fluctuating with short cycles and a relatively small amplitude, as shown with the first signal waveforms for the unit periods T1 to T6 and T8 to T10 in FIGs. 6 to 13. The AC signal 520 affected by scattered light from foreign matter has a waveform resulting from superimposing a waveform having short-cycle fluctuations on a waveform having slow and large changes, as shown with the first signal waveform for the unit period T7 in FIGs. 6 to 13. The AC values thus tend to have a higher standard deviation for the AC signal 520 affected by scattered light from foreign matter than for the AC signal 520 unaffected by scattered light from foreign matter.

In the present example, as described above, the identifier 500 determines whether the first signal portion for the target unit period T is an abnormal signal portion based on the standard deviation $\sigma u$ corresponding to the first signal waveform for the target unit period T. More specifically, the identifier 500 compares the standard deviation $\sigma u$ corresponding to the first signal waveform for the target unit period T with the average $\sigma u$ave of the standard deviations $\sigma u$ for M unit periods T preceding the target unit period T. The identifier 500 uses the comparison result to determine whether the first signal portion for the target unit period T is an abnormal signal portion. For example, the identifier 500 calculates the ratio MG of the standard deviation $\sigma u$ to the average $\sigma u$ave. When the calculated ratio MG is greater than or equal to the first threshold, the identifier 500 determines that the target first signal portion is an abnormal signal portion. To allow the identifier 500 to appropriately determine whether the first signal portion is an abnormal signal portion, the unit period T may have the length defined based on, for example, the signal waveform of the AC signal 520 obtained through preliminary experimentation using foreign matter that is likely to occur.

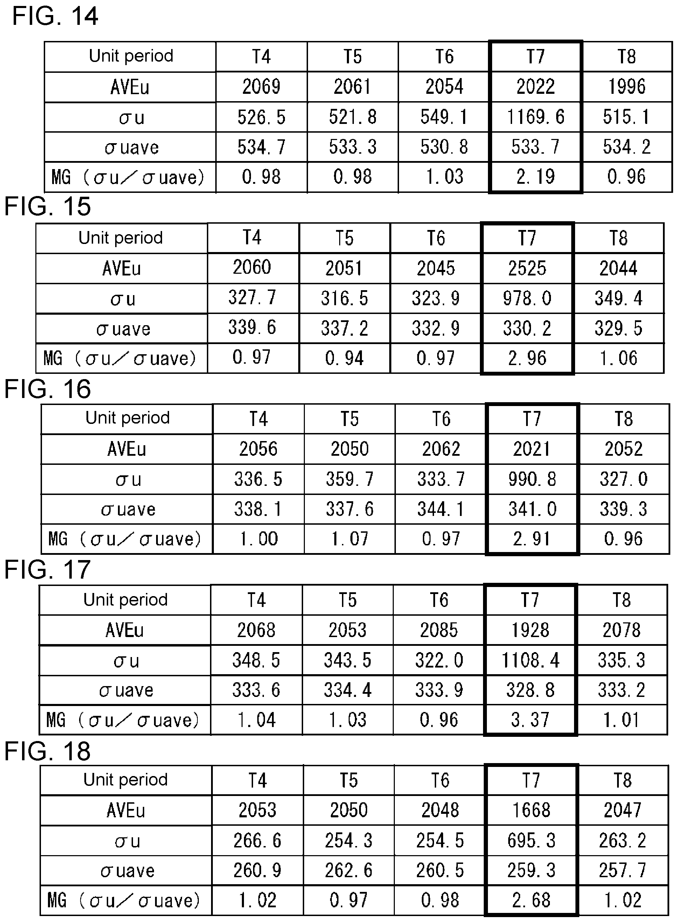

FIGs. 14 to 21 are tables showing the calculation results of the standard deviation $\sigma u$, the average $\sigma u$ave, and the ratio MG for the signal waveforms shown in FIGs. 6 to 13. FIGs. 14 to 21 each show the average $\sigma u$ave obtained when M = 5. Further, FIGs. 14 to 21 each show an average AVEu of the first signal waveform for the unit period T. The average AVEu may be the average of the N AC values included in the first signal portion.

In FIGs. 14 to 21, the column for each unit period T includes the average AVEu and the standard deviation $\sigma u$ corresponding to the first signal waveform for the unit period T. The column for each unit period T also includes the average $\sigma u$ave as a reference of comparison with the standard deviation $\sigma u$ corresponding to the first signal waveform for the unit period T. The column for each unit period T also includes the ratio MG of the standard deviation $\sigma u$ corresponding to the first signal waveform to the average $\sigma u$ave for the unit period T. The ratio MG of the standard deviation $\sigma u$ corresponding to the first signal waveform to the average $\sigma u$ave for the unit period T is hereafter referred to as the ratio MG for the unit period T.

As shown in FIGs. 14 to 21, under constant conditions such as the flow rate of the fluid 802 and the light quantity of the light emitter 30, the first signal waveforms have substantially constant standard deviations $\sigma u$ for the unit periods T for which no scattered light from foreign matter is received by the light receiver 31 (the unit periods T4 to T6 and T8 in FIGs. 14 to 21). In other words, the first signal waveforms have substantially constant standard deviations $\sigma u$ when unaffected by scattered light from foreign matter. Thus, under constant conditions such as the flow rate of the fluid 802 and the light quantity of the light emitter 30, the first signal waveforms in M normal unit periods T have the standard deviations $\sigma u$ with a substantially constant average $\sigma u$ave. For each unit period T for which no scattered light from foreign matter is received by the light receiver 31, the ratio MG for the unit period T is close to 1.0.

In contrast, the first signal waveforms have higher standard deviations $\sigma u$ for the unit periods T with scattered light from foreign matter received by the light receiver 31 (the unit period T7 in FIGs. 14 to 21) than without scattered light from foreign matter. In other words, the first signal waveforms have higher standard deviations $\sigma u$ when affected by scattered light from foreign matter than when unaffected by scattered light from foreign matter. For the unit period T for which scattered light from foreign matter is received by the light receiver 31, the ratio MG for the unit period T is greater than 1.0. In the examples of FIGs. 14 to 21, the ratio MG for the unit period T is greater than 2.0 for the unit period T for which scattered light from foreign matter is received by the light receiver 31.

The above first threshold used by the identifier 500 in step s3 is defined based on, for example, the ratio MG obtained through preliminary experimentation using foreign matter that is likely to occur. For the ratios MG shown in FIG. 14 obtained through preliminary experimentation, for example, the first threshold may be set to 1.5. In this case, for example, the identifier 500 determines that the first signal portion for the target unit period T is an abnormal signal portion when the ratio MG calculated for the target unit period T is greater than or equal to 1.5 in the first determination. For the ratios MG shown in FIGs. 15 to 21 obtained through preliminary experimentation, the first threshold is set to, for example, 1.5.

The identifier 500 may perform the first determination without calculating the ratio MG. More specifically, the identifier 500 may determine that the target first signal portion is an abnormal signal portion when the first signal waveform for the target unit period T has the standard deviation $\sigma u$ greater than or equal to a second threshold or is greater than the second threshold. For example, for the standard deviations $\sigma u$ shown in FIG. 14 obtained through preliminary experimentation, the second threshold is set to, for example, 800. For the standard deviations $\sigma u$ shown in FIGs. 15 to 21 obtained through preliminary experimentation, the second threshold is set to, for example, 500.

As described above, the identifier 500 determines whether the first signal portion for the target unit period T is an abnormal signal portion based on the standard deviation $\sigma u$ corresponding to the first signal waveform for the target unit period T. The identifier 500 can thus identify an abnormal signal portion of the AC signal 520 with increased accuracy. The calculator 501 can thus appropriately calculate the flow state value Va based on the signal portions of the AC signal 520 other than the abnormal signal portion. This allows the flow state value Va to be calculated with increased accuracy.

In the measurement device 1, the fluid 802 flowing at a lower flow rate tends to cause the AC signal 520 to have a signal waveform with greater amplitude fluctuations. The fluid 802 flowing at a lower flow rate has a narrower distribution range of the flow velocity in the flow path component 801 (in other words, a smaller variation in the flow velocity in the flow path component 801). This causes the AC signal 520 to be a combination of multiple AC signal components with frequencies close to each other. For the AC signal 520 having a signal waveform with greater amplitude fluctuations, the first signal waveform for each unit period T has a higher standard deviation σu.

For example, the signal waveform has greater amplitude fluctuations for the fluid 802 flowing at a flow rate of 20 ml/min shown in FIGs. 7 to 9 than for the fluid 802 flowing at a flow rate of 50 ml/min shown in FIGs. 10 to 13. The standard deviations σu are thus higher for the fluid 802 flowing at a flow rate of 20 ml/min shown in FIGs. 15 to 17 than for the fluid 802 flowing at a flow rate of 50 ml/min shown in FIGs. 18 to 21.

With the standard deviation σu changing in accordance with a change in the flow rate of the fluid 802, the identifier 500 may erroneously determine that a normal target first signal portion is an abnormal signal portion when comparing the standard deviation σu corresponding to the first signal portion waveform for the target unit period T with a fixed second threshold and determining whether the target first signal portion is an abnormal signal portion based on the comparison result.

With the standard deviation σu changing in accordance with a change in the flow rate of the fluid 802, the average σuave also changes accordingly. The identifier 500 can thus determine whether the target first signal portion is an abnormal signal portion more appropriately based on the result of comparison between the standard deviation σu and the average σuave for the target unit period T. This allows the flow state value Va to be calculated with increased accuracy.

Second Example

In the abnormality determination in the present example, the identifier 500 calculates a difference R between a maximum value MAX and a minimum value MIN of the target first signal waveform in step s2 for the target unit period T. The difference R may be a difference between the maximum value MAX and the minimum value MIN of the N AC values included in the target first signal portion. The identifier 500 uses the calculated difference R as the characteristic quantity of the target first signal waveform.

In step s3 for the target unit period T, the identifier 500 performs a process of determining whether the target first signal portion is an abnormal signal portion based on the difference R (also referred to as second determination). In the second determination, when, for example, the difference R is greater than or equal to a third threshold, the identifier 500 determines that the target first signal portion is an abnormal signal portion. When the difference R is less than the third threshold, the identifier 500 determines that the target first signal portion is not an abnormal signal portion. The identifier 500 may determine that the target first signal portion is an abnormal signal portion when the difference R is greater than the third threshold, and may determine that the target first signal portion is not an abnormal signal portion when the difference R is less than or equal to the third threshold.

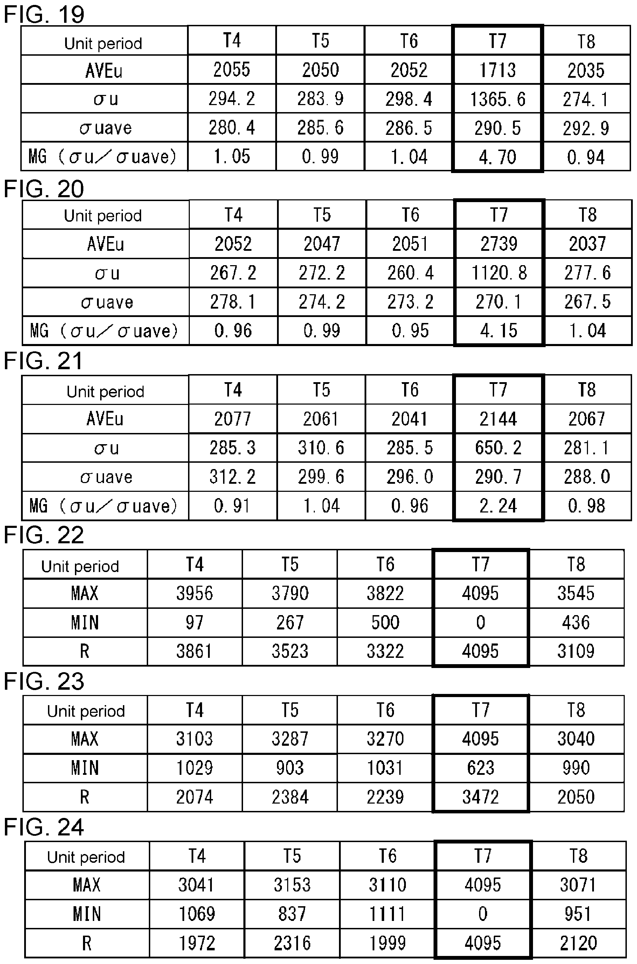

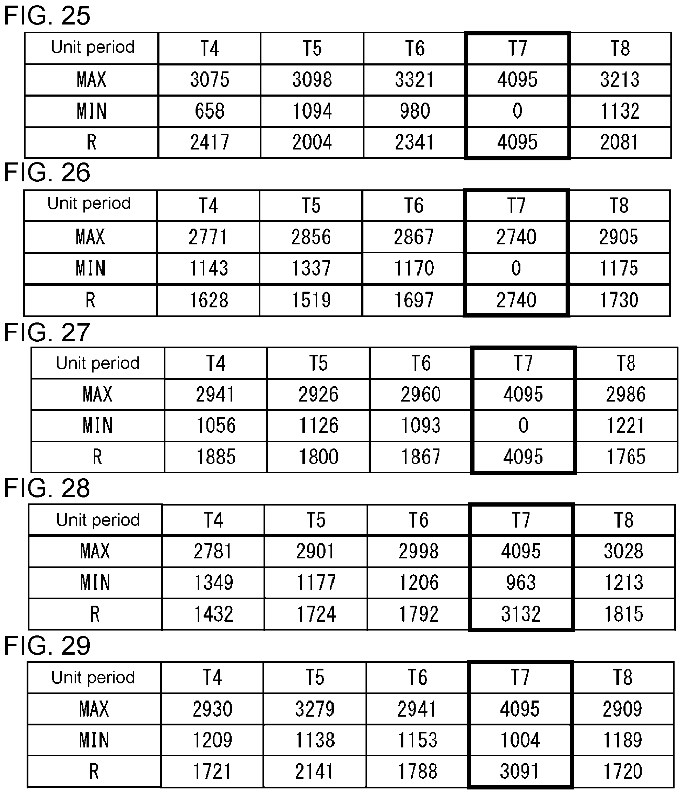

FIGs. 22 to 29 are tables showing the calculation results of the maximum value MAX, the minimum value MIN, and the difference R for the signal waveforms shown in FIGs. 6 to 13. In FIGs. 22 to 29, the column for each unit period T includes the maximum value MAX, the minimum value MIN, and the difference R between the maximum value MAX and the minimum value MIN for the first signal waveform for the unit period T.

As described above, the AC signal 520 affected by scattered light from foreign matter has a waveform obtained by superimposing a waveform having short-cycle fluctuations on a waveform having slow and large changes. For each of the unit periods T for which scattered light from foreign matter is received by the light receiver 31 (the unit period T7 in FIGs. 22 to 29), the first signal waveform has a greater difference R between the maximum value MAX and the minimum value MIN.

Thus, as described above, the identifier 500 uses the difference R to determine whether the target first signal portion is an abnormal signal portion. This allows appropriate determination as to whether the target first signal portion is an abnormal signal portion.

The above third threshold used by the identifier 500 is defined based on, for example, the difference R obtained through preliminary experimentation using foreign matter that is likely to occur. For example, for the difference R shown in FIG. 22 obtained through preliminary experimentation, the third threshold is set to, for example, 3900. For the differences R shown in FIGs. 23 to 29 obtained through preliminary experimentation, the third threshold is set to, for example, 2600.

Third Example

In the abnormality determination in the present example, the identifier 500 divides the target unit period T into L subperiods PT in step s2 for the target unit period T. L is an integer greater than or equal to 2. Each subperiod PT has a length 1/L of the length of the unit period T.

The identifier 500 then calculates, for each of the L subperiods PT, an average AVEp of the signal waveforms (second signal waveforms) of the signal portions (also referred to as second signal portions) for the subperiod PT contained in the target first signal portion. The average AVEp may be the average of multiple AC values included in the second signal portion. The identifier 500 calculates a standard deviation $\sigma$ap of the L averages AVEp calculated for the L subperiods PT. The identifier 500 uses the calculated standard deviation $\sigma$ap as the characteristic quantity of the target first signal waveform.

The number of AC values included in the second signal portion is set to a value calculated by, for example, dividing N by L, where N is the number of multiple AC values included in the first signal portion. When N = 1024 and L = 4, for example, the number of AC values included in the second signal portion is 256.

In step s3 for the target unit period T, the identifier 500 performs a process of determining whether the target first signal portion is an abnormal signal portion based on the standard deviation $\sigma$ap (also referred to as third determination). In the third determination, the identifier 500 calculates, for example, the average AVEap of the L averages AVEp calculated for the L subperiods PT in step s2. The identifier 500 calculates a variation coefficient CV for the average AVEp based on the standard deviation $\sigma$ap and the average AVEap. The variation coefficient CV is calculated by dividing the standard deviation $\sigma$ap by the average AVEap.

In the third determination, the identifier 500 calculates a variation coefficient CV and compares the variation coefficient CV with a fourth threshold. The identifier 500 uses the comparison result to determine whether the target first signal portion is an abnormal signal portion. For example, when the variation coefficient CV is greater than or equal to the fourth threshold, the identifier 500 determines that the target first signal portion is an abnormal signal portion. When the variation coefficient CV is less than the fourth threshold, the identifier 500 determines that the target first signal portion is not an abnormal signal portion. The identifier 500 may determine that the target first signal portion is an abnormal signal portion when the variation coefficient CV is greater than the fourth threshold, and may determine that the target first signal portion is not an abnormal signal portion when the variation coefficient CV is less than or equal to the fourth threshold.

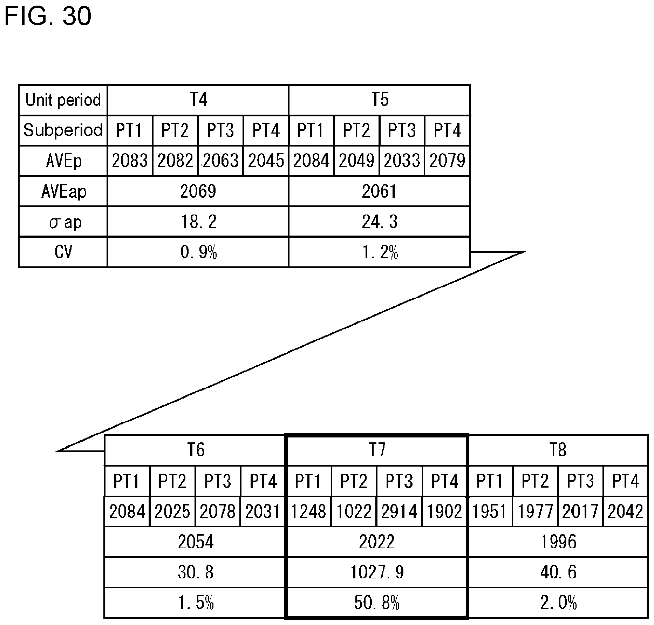

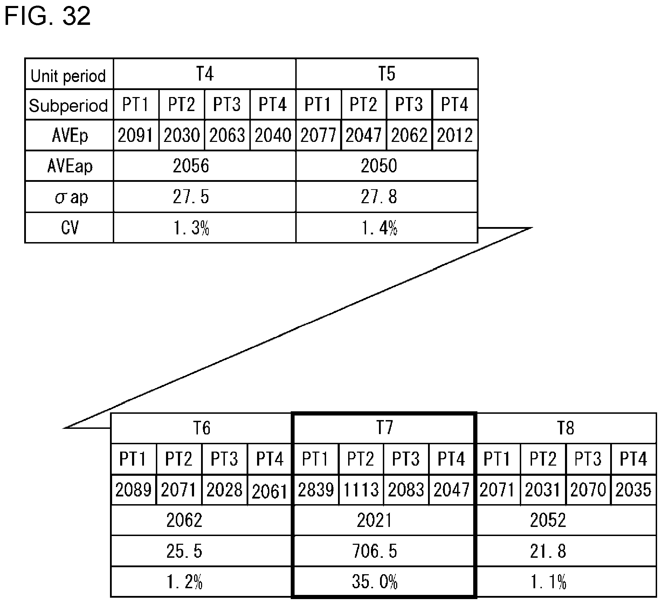

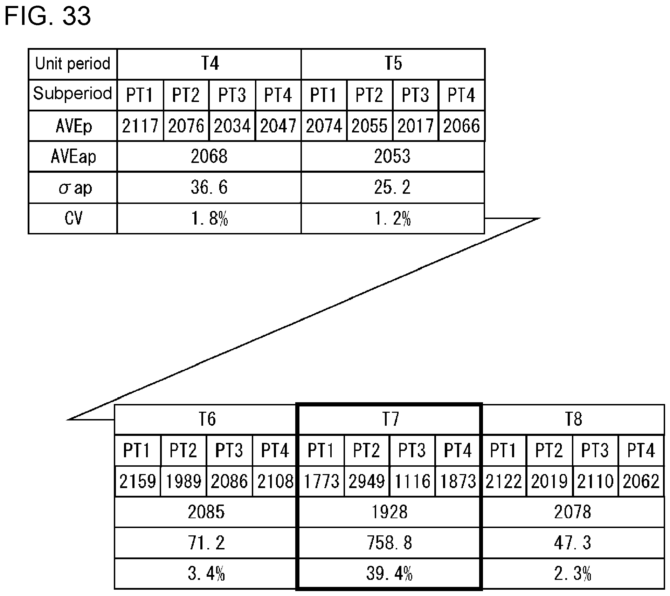

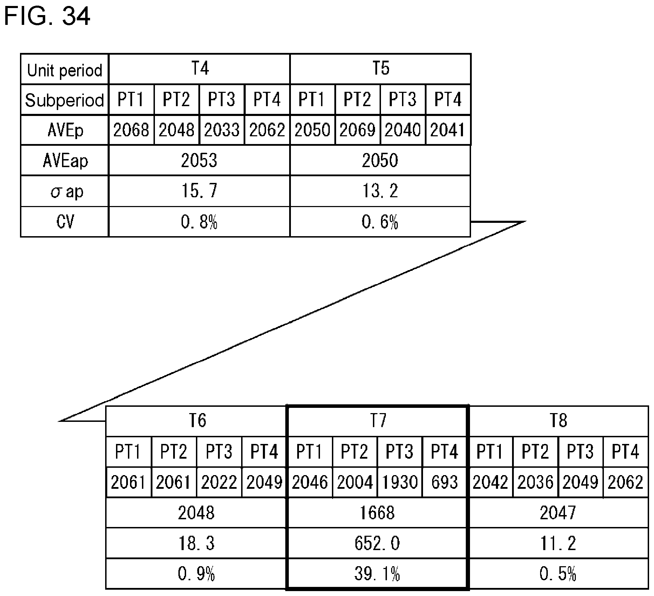

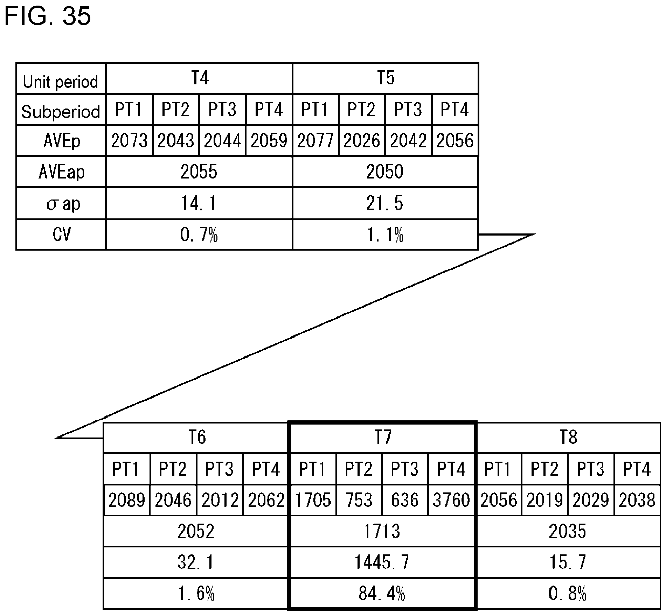

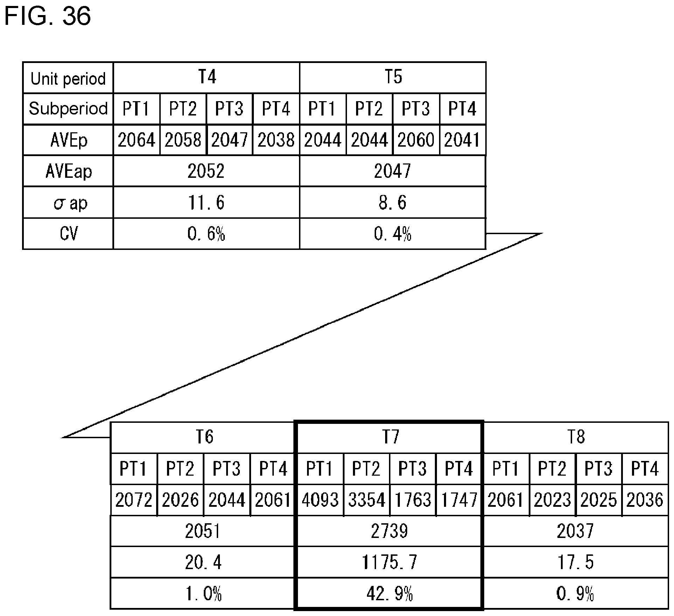

FIGs. 30 to 37 are tables showing the calculation results of the average AVEp, the average AVEap, the standard deviation $\sigma$ap, and the variation coefficient CV for the signal waveforms shown in FIGs. 6 to 13. In the examples of FIGs. 30 to 37, N = 1024, L = 4, and the number of AC values included in the second signal portion is 256. In FIGs. 30 to 37, the column for each unit period T includes the averages AVEp of the second signal waveforms for the respective four subperiods PTs, or PT1, PT2, PT3, and PT4. The column for each unit period T also includes the average AVEap of the four averages AVEp for the four subperiods PTs, or PT1, PT2, PT3, and PT4, included in the unit period T, the standard deviation $\sigma$ap of the four averages AVEp, and the variation coefficient CV based on the average AVEap and standard deviation $\sigma$ap. In FIGs. 30 to 37, the variation coefficients CV are expressed as percentages. In other words, each variation coefficient CV in FIGs. 30 to 37 is calculated by dividing the standard deviation $\sigma$ap by the average AVEap and multiplying the resultant by 100.

Unlike the AC signal 520 affected by scattered light from foreign matter, the AC signal 520 unaffected by scattered light from foreign matter changes in a manner different from slow and large changes. The average AVEp thus varies less widely among the subperiods PT included in the unit periods T for which no scattered light from foreign matter is received by the light receiver 31 (the unit periods T4 to T6 and T8 in FIGs. 30 to 37). Thus, the standard deviation $\sigma$ap and the variation coefficient CV are smaller for the unit periods T for which no scattered light from foreign matter is received by the light receiver 31.

In contrast, the AC signal 520 affected by scattered light from the foreign matter changes slowly and largely. The average AVEp thus varies more widely among the subperiods PT included in the unit periods T for which scattered light from foreign matter is received by the light receiver 31 (the unit period T7 in FIGs. 30 to 37). Thus, the standard deviation $\sigma$ap and the variation coefficient CV are greater for the unit periods T for which scattered light from foreign matter is received by the light receiver 31.

Thus, as described above, the identifier 500 uses the variation coefficient CV calculated for the target unit period T to determine whether the target first signal portion is an abnormal signal portion. This allows appropriate determination as to whether the target first signal portion is an abnormal signal portion.

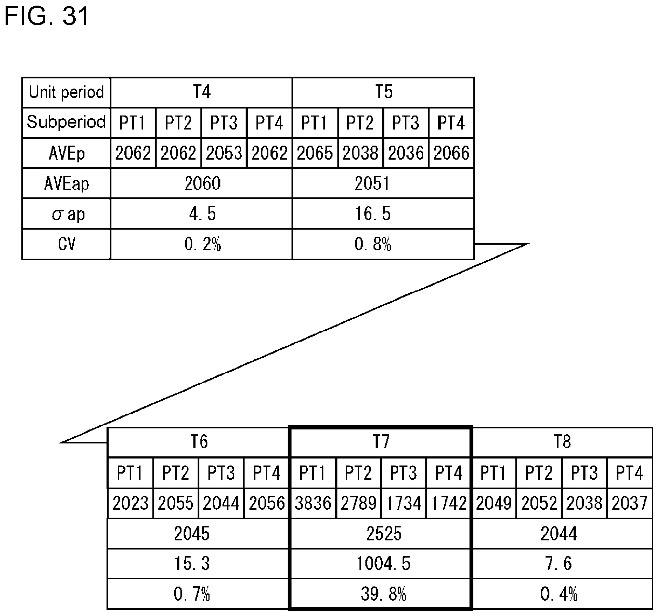

The above fourth threshold used by the identifier 500 is defined based on, for example, the variation coefficient CV obtained through preliminary experimentation using foreign matter that is likely to occur. For example, for the variation coefficients CV shown in FIG. 30 obtained through preliminary experimentation, the fourth threshold is set to, for example, 5%. For the variation coefficients CV shown in FIGs. 31 to 37 obtained through preliminary experimentation, the fourth threshold is set to, for example, 8%.

The identifier 500 may perform the third determination without calculating the variation coefficient CV More specifically, the identifier 500 may determine that the target first signal portion is an abnormal signal portion when the standard deviation $\sigma$ap for the target unit period T is greater than or equal to a fifth threshold or is greater than the fifth threshold. For example, for the standard deviations $\sigma$ap shown in FIG. 30 obtained through preliminary experimentation, the fifth threshold is set to, for example, 500. For the standard deviations $\sigma$ap shown in FIGs. 31 to 37 obtained through preliminary experimentation, the fifth threshold is set to, for example, 200.

In the example of FIG. 4 above, steps s1 to s4 are performed continuously. However, another process may be performed between steps s3 and s4. The processing in steps s1 to s3 may be performed separately from and independently of the processing in step s4. In this case, in step s4, the flow state value Va may be calculated by a discrete Fourier transform on more or less than N of time-series normal AC values. The characteristic quantity and the flow state value Va may be calculated using different numbers of AC values.

The characteristic quantities used in the abnormality determination may include the standard deviation σu in the first example and the difference R in the second example. In this case, for example, in step s3, the first determination using the standard deviation σu and the second determination using the difference R are performed. When the target first signal portion is determined to be an abnormal signal portion in at least one of the first determination or the second determination, step s1 is performed again without performing step s4. When the target first signal portion is determined not to be an abnormal signal portion in either the first determination or the second determination, step s4 is performed.

The characteristic quantities used in the abnormality determination may include the standard deviation σu in the first example and the standard deviation σap in the third example. In this case, for example, in step s3, the first determination using the standard deviation σu and the third determination using the standard deviation σap are performed. When the target first signal portion is determined to be an abnormal signal portion in at least one of the first determination or the third determination, step s1 is performed again without performing step s4. When the target first signal portion is determined not to be an abnormal signal portion in either the first determination or the third determination, step s4 is performed.

The characteristic quantities used in the abnormality determination may include the difference R and the standard deviation σap. In this case, for example, in step s3, the second determination and the third determination are performed. When the target first signal portion is determined to be an abnormal signal portion in at least one of the second determination or the third determination, step s1 is performed again without performing step s4. When the target first signal portion is determined not to be an abnormal signal portion in either the second determination or the third determination, step s4 is performed.

The characteristic quantities used in the abnormality determination may include the standard deviation σu, the difference R, and the standard deviation σap. In this case, for example, in step s3, the first determination, the second determination, and the third determination are performed. When the target first signal portion is determined to be an abnormal signal portion in at least one of the first determination, the second determination, or the third determination, step s1 is performed again without performing step s4. When the target first signal portion is determined not to be an abnormal signal portion in any of the first determination, the second determination, or the third determination, step s4 is performed.

In response to an abnormal signal portion of the AC signal 520 identified by the identifier 500, the measurement device 1 may output a notification signal (also referred to as a foreign matter notification signal) to provide information about foreign matter contained in the fluid 802 to a device external to the measurement device 1. In this case, for example, the arithmetic circuit 5 generates a foreign matter notification signal when the target first signal portion is determined to be an abnormal signal portion in step s3. The arithmetic circuit 5 then outputs the generated foreign matter notification signal to a device external to the measurement device 1 through the connector 6. The foreign matter notification signal is input from the measurement device 1 into the external device connected to the connector 6. This allows the external device to receive information about foreign matter having been identified in the fluid 802 in the measurement device 1.

The measurement device 1 may include a real-time clock for outputting the current time. In this case, the measurement device 1 may output, to a device external to the measurement device 1, foreign matter notification information including the time at which foreign matter is identified (also referred to as a foreign matter identification time). This allows the external device connected to the connector 6 to receive information about the time at which foreign matter is identified in the measurement device 1. The measurement device 1 may use, as the foreign matter identification time, the time at which the target first signal portion is determined to be an abnormal signal portion in step s3.

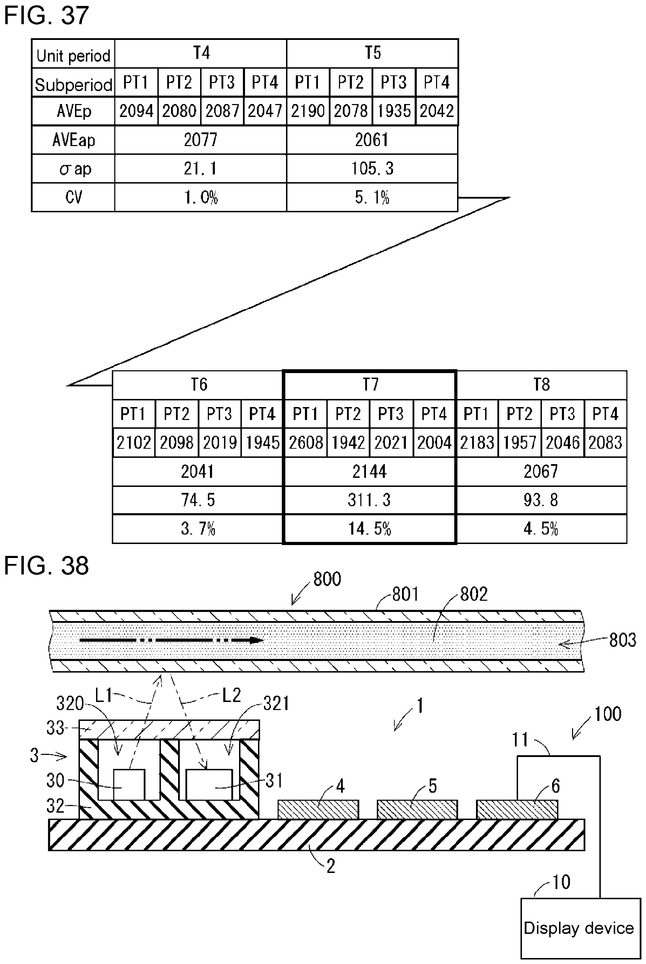

The external device connected to the measurement device 1 may be, for example, a display device. FIG. 38 is a diagram of an example measurement system 100 including the measurement device 1 and a display device 10 connected to the measurement device 1. The external device may be a device other than a display device.

In the example of FIG. 38, the display device 10 is connected to the connector 6 with a cable 11. The display device 10 displays, for example, various items of information such as letters, symbols, and graphics. The display device 10 may display various items of information about the measurement of flow of the fluid 802 in the measurement device 1. The display device 10 may be, for example, a liquid crystal display device or another display device.

The display device 10 may display the flow state value Va calculated by the arithmetic circuit 5 and received through the connector 6. In this case, the display device 10 may display the calculation value Vb included in the flow state value Va, the flow rate included in the flow state value Va, or the flow velocity included in the flow state value Va.

In response to an abnormality notification signal from the arithmetic circuit 5 through the connector 6, the display device 10 may display notification information indicating that the fluid 802 contains foreign matter (also referred to as foreign matter notification). This allows a user to learn, from the display device 10, that the fluid 802 contains foreign matter. The display device 10 may display the foreign matter notification information in text, graphics, or a combination of text and graphics. The display device 10 may display a foreign matter identification time in response to a foreign matter notification signal containing the foreign matter identification time from the arithmetic circuit 5 through the connector 6. This allows a user to learn, from the display device 10, the time at which foreign matter in the fluid 802 is identified in the measurement device 1.

The measurement device 1 may be connected to an external device, such as the display device 10, wirelessly instead of using wires. In this case, the measurement device 1 may replace the connector 6 with a communication circuit for wireless communication with an external device.

The measurement device 1 and the measurement system 100 have been described in detail as above, but the foregoing structures are illustrative in all respects, and the disclosure is not limited to the above structures. All the features of the embodiments described above may be combined in use unless any contradiction arises. Many variations not specifically described above may be implemented without departing from the scope of the disclosure.

REFERENCE SIGNS

| 1 | measurement device |
| 10 | display device |
| 30 | light emitter |
| 31 | light receiver |
| 100 | measurement system |
| 500 | identifier |
| 501 | calculator |
| 510 | program |
| 800 | irradiation target |

**Claims**

1. A measurement device, comprising:

a light emitter configured to irradiate, with light, an irradiation target having a fluid flowing in an internal space of the irradiation target; a light receiver configured to receive coherent light including light scattered by the irradiation target and output an output signal corresponding to an intensity of the coherent light; an identifier configured to obtain a characteristic quantity indicating a characteristic of a signal waveform of the output signal and identify, based on the obtained characteristic quantity, an abnormal signal portion of the output signal; and a calculator configured to calculate, based on a signal portion of the output signal other than the abnormal signal portion, a flow state value indicating a flow state of the fluid.

2. The measurement device according to claim 1, wherein the identifier periodically obtains, for a first period, the characteristic quantity for a first signal waveform of a first signal portion of the output signal and determines, based on the obtained characteristic quantity, whether the first signal portion is the abnormal signal portion.

3. The measurement device according to claim 2, wherein the characteristic quantity includes a difference between a maximum value and a minimum value of the first signal waveform.

4. The measurement device according to claim 2 or claim 3, wherein the characteristic quantity includes a first standard deviation for the first signal waveform.

5. The measurement device according to claim 4, wherein the identifier compares the first standard deviation corresponding to a target first period with a first average of first standard deviations corresponding to a plurality of the first periods preceding the target first period and determines, based on a result of the comparison, whether the first signal portion for the target first period is the abnormal signal portion.

6. The measurement device according to any one of claims 2 to 5, wherein

the first period includes a plurality of second periods, the identifier periodically calculates a second average for a second signal waveform of a second signal portion of the first signal portion for each of the plurality of second periods and calculates a second standard deviation of the calculated second average corresponding to each of the plurality of second periods, and the characteristic quantity includes the second standard deviation.

7. The measurement device according to claim 6, wherein the identifier determines whether the first signal portion is the abnormal signal portion based on a variation coefficient resulting from a division of the second standard deviation by an average of the calculated second average corresponding to each of the plurality of second periods.

8. The measurement device according to any one of claims 1 to 7, wherein
in response to the abnormal signal portion identified by the identifier, the measurement device outputs, to a device external to the measurement device, a notification signal indicating that the fluid contains foreign matter.

9. A measurement system, comprising:

the measurement device according to any one of claims 1 to 8; and
a display device configured to display the flow state value calculated by the measurement device.

10. A measurement system, comprising:

the measurement device according to claim 8; and
a display device configured to display, in response to the notification signal output from the measurement device to the device external to the measurement device, notification information indicating that the fluid contains foreign matter.

11. A measurement method implementable with a device, the method comprising:

obtaining a characteristic quantity indicating a characteristic of a signal waveform of an output signal corresponding to an intensity of coherent light output from a light receiver receiving the coherent light, the coherent light including light scattered by an irradiation target irradiated with light by a light emitter, the irradiation target having a fluid flowing in an internal space of the irradiation target, and identifying, based on the obtained characteristic quantity, an abnormal signal portion of the output signal; and
calculating, based on a signal portion of the output signal other than the abnormal signal portion, a flow state value indicating a flow state of the fluid.

12. A program executable by a computer to receive an input of an output signal corresponding to an intensity of coherent light output from a light receiver receiving the coherent light, the coherent light including light scattered by an irradiation target irradiated with light by a light emitter, the irradiation target having a fluid flowing in an internal space of the irradiation target, the program causing the computer to perform operations comprising:

obtaining a characteristic quantity indicating a characteristic of a signal waveform of the output signal, and identifying, based on the obtained characteristic quantity, an abnormal signal portion of the output signal; and
calculating, based on a signal portion of the output signal other than the abnormal signal portion, a flow state value indicating a flow state of the fluid.

# FIG. 1

# FIG. 2

# FIG. 3

FIG. 4

```
                              ┌─ s1
        ┌──────────────────────────────┐
        │  Obtain first AC values included in │
        │       first signal portion          │
        └──────────────────────────────┘
                       │
                       ▼       ┌─ s2
        ┌──────────────────────────────┐
        │   Obtain characteristic quantity    │
        └──────────────────────────────┘
                       │
                       ▼           ┌─ s3
        ╱──────────────────────────────╲        Yes
       ⟨    First signal portion abnormal?    ⟩ ────→
        ╲──────────────────────────────╱
                       │ No
                       ▼       ┌─ s4
        ┌──────────────────────────────┐
        │     Calculate flow state value      │
        └──────────────────────────────┘
```

FIG. 5

Signal strength vs Frequency

FIG. 6

EP 4 220 187 A1

FIG. 7

FIG. 8

EP 4 220 187 A1

FIG. 9

FIG. 10

# FIG. 11

EP 4 220 187 A1

FIG. 12

FIG. 13

## FIG. 14

| Unit period | T4 | T5 | T6 | T7 | T8 |
|---|---|---|---|---|---|
| AVEu | 2069 | 2061 | 2054 | 2022 | 1996 |
| σu | 526.5 | 521.8 | 549.1 | 1169.6 | 515.1 |
| σuave | 534.7 | 533.3 | 530.8 | 533.7 | 534.2 |
| MG (σu/σuave) | 0.98 | 0.98 | 1.03 | 2.19 | 0.96 |

## FIG. 15

| Unit period | T4 | T5 | T6 | T7 | T8 |
|---|---|---|---|---|---|
| AVEu | 2060 | 2051 | 2045 | 2525 | 2044 |
| σu | 327.7 | 316.5 | 323.9 | 978.0 | 349.4 |
| σuave | 339.6 | 337.2 | 332.9 | 330.2 | 329.5 |
| MG (σu/σuave) | 0.97 | 0.94 | 0.97 | 2.96 | 1.06 |

## FIG. 16

| Unit period | T4 | T5 | T6 | T7 | T8 |
|---|---|---|---|---|---|
| AVEu | 2056 | 2050 | 2062 | 2021 | 2052 |
| σu | 336.5 | 359.7 | 333.7 | 990.8 | 327.0 |
| σuave | 338.1 | 337.6 | 344.1 | 341.0 | 339.3 |
| MG (σu/σuave) | 1.00 | 1.07 | 0.97 | 2.91 | 0.96 |

## FIG. 17

| Unit period | T4 | T5 | T6 | T7 | T8 |
|---|---|---|---|---|---|
| AVEu | 2068 | 2053 | 2085 | 1928 | 2078 |
| σu | 348.5 | 343.5 | 322.0 | 1108.4 | 335.3 |
| σuave | 333.6 | 334.4 | 333.9 | 328.8 | 333.2 |
| MG (σu/σuave) | 1.04 | 1.03 | 0.96 | 3.37 | 1.01 |

## FIG. 18

| Unit period | T4 | T5 | T6 | T7 | T8 |
|---|---|---|---|---|---|
| AVEu | 2053 | 2050 | 2048 | 1668 | 2047 |
| σu | 266.6 | 254.3 | 254.5 | 695.3 | 263.2 |
| σuave | 260.9 | 262.6 | 260.5 | 259.3 | 257.7 |
| MG (σu/σuave) | 1.02 | 0.97 | 0.98 | 2.68 | 1.02 |

## FIG. 19

| Unit period | T4 | T5 | T6 | T7 | T8 |
|---|---|---|---|---|---|
| AVEu | 2055 | 2050 | 2052 | 1713 | 2035 |
| $\sigma$ u | 294.2 | 283.9 | 298.4 | 1365.6 | 274.1 |
| $\sigma$ uave | 280.4 | 285.6 | 286.5 | 290.5 | 292.9 |
| MG ($\sigma$ u／$\sigma$ uave) | 1.05 | 0.99 | 1.04 | 4.70 | 0.94 |

## FIG. 20

| Unit period | T4 | T5 | T6 | T7 | T8 |
|---|---|---|---|---|---|
| AVEu | 2052 | 2047 | 2051 | 2739 | 2037 |
| $\sigma$ u | 267.2 | 272.2 | 260.4 | 1120.8 | 277.6 |
| $\sigma$ uave | 278.1 | 274.2 | 273.2 | 270.1 | 267.5 |
| MG ($\sigma$ u／$\sigma$ uave) | 0.96 | 0.99 | 0.95 | 4.15 | 1.04 |

## FIG. 21

| Unit period | T4 | T5 | T6 | T7 | T8 |
|---|---|---|---|---|---|
| AVEu | 2077 | 2061 | 2041 | 2144 | 2067 |
| $\sigma$ u | 285.3 | 310.6 | 285.5 | 650.2 | 281.1 |
| $\sigma$ uave | 312.2 | 299.6 | 296.0 | 290.7 | 288.0 |
| MG ($\sigma$ u／$\sigma$ uave) | 0.91 | 1.04 | 0.96 | 2.24 | 0.98 |

## FIG. 22

| Unit period | T4 | T5 | T6 | T7 | T8 |
|---|---|---|---|---|---|
| MAX | 3956 | 3790 | 3822 | 4095 | 3545 |
| MIN | 97 | 267 | 500 | 0 | 436 |
| R | 3861 | 3523 | 3322 | 4095 | 3109 |

## FIG. 23

| Unit period | T4 | T5 | T6 | T7 | T8 |
|---|---|---|---|---|---|
| MAX | 3103 | 3287 | 3270 | 4095 | 3040 |
| MIN | 1029 | 903 | 1031 | 623 | 990 |
| R | 2074 | 2384 | 2239 | 3472 | 2050 |

## FIG. 24

| Unit period | T4 | T5 | T6 | T7 | T8 |
|---|---|---|---|---|---|
| MAX | 3041 | 3153 | 3110 | 4095 | 3071 |
| MIN | 1069 | 837 | 1111 | 0 | 951 |
| R | 1972 | 2316 | 1999 | 4095 | 2120 |

## FIG. 25

| Unit period | T4 | T5 | T6 | T7 | T8 |
|---|---|---|---|---|---|
| MAX | 3075 | 3098 | 3321 | 4095 | 3213 |
| MIN | 658 | 1094 | 980 | 0 | 1132 |
| R | 2417 | 2004 | 2341 | 4095 | 2081 |

## FIG. 26

| Unit period | T4 | T5 | T6 | T7 | T8 |
|---|---|---|---|---|---|
| MAX | 2771 | 2856 | 2867 | 2740 | 2905 |
| MIN | 1143 | 1337 | 1170 | 0 | 1175 |
| R | 1628 | 1519 | 1697 | 2740 | 1730 |

## FIG. 27

| Unit period | T4 | T5 | T6 | T7 | T8 |
|---|---|---|---|---|---|
| MAX | 2941 | 2926 | 2960 | 4095 | 2986 |
| MIN | 1056 | 1126 | 1093 | 0 | 1221 |
| R | 1885 | 1800 | 1867 | 4095 | 1765 |

## FIG. 28

| Unit period | T4 | T5 | T6 | T7 | T8 |
|---|---|---|---|---|---|
| MAX | 2781 | 2901 | 2998 | 4095 | 3028 |
| MIN | 1349 | 1177 | 1206 | 963 | 1213 |
| R | 1432 | 1724 | 1792 | 3132 | 1815 |

## FIG. 29

| Unit period | T4 | T5 | T6 | T7 | T8 |
|---|---|---|---|---|---|
| MAX | 2930 | 3279 | 2941 | 4095 | 2909 |
| MIN | 1209 | 1138 | 1153 | 1004 | 1189 |
| R | 1721 | 2141 | 1788 | 3091 | 1720 |

FIG. 30

| Unit period | T4 | | | | T5 | | | |
|---|---|---|---|---|---|---|---|---|
| Subperiod | PT1 | PT2 | PT3 | PT4 | PT1 | PT2 | PT3 | PT4 |
| AVEp | 2083 | 2082 | 2063 | 2045 | 2084 | 2049 | 2033 | 2079 |
| AVEap | 2069 | | | | 2061 | | | |
| $\sigma$ ap | 18.2 | | | | 24.3 | | | |
| CV | 0.9% | | | | 1.2% | | | |

| | T6 | | | | T7 | | | | T8 | | | |
|---|---|---|---|---|---|---|---|---|---|---|---|---|
| | PT1 | PT2 | PT3 | PT4 | PT1 | PT2 | PT3 | PT4 | PT1 | PT2 | PT3 | PT4 |
| | 2084 | 2025 | 2078 | 2031 | 1248 | 1022 | 2914 | 1902 | 1951 | 1977 | 2017 | 2042 |
| | 2054 | | | | 2022 | | | | 1996 | | | |
| | 30.8 | | | | 1027.9 | | | | 40.6 | | | |
| | 1.5% | | | | 50.8% | | | | 2.0% | | | |

FIG. 31

| Unit period | T4 | | | | T5 | | | |
|---|---|---|---|---|---|---|---|---|
| Subperiod | PT1 | PT2 | PT3 | PT4 | PT1 | PT2 | PT3 | PT4 |
| AVEp | 2062 | 2062 | 2053 | 2062 | 2065 | 2038 | 2036 | 2066 |
| AVEap | 2060 | | | | 2051 | | | |
| $\sigma$ ap | 4. 5 | | | | 16. 5 | | | |
| CV | 0. 2% | | | | 0. 8% | | | |

| T6 | | | | T7 | | | | T8 | | | |
|---|---|---|---|---|---|---|---|---|---|---|---|
| PT1 | PT2 | PT3 | PT4 | PT1 | PT2 | PT3 | PT4 | PT1 | PT2 | PT3 | PT4 |
| 2023 | 2055 | 2044 | 2056 | 3836 | 2789 | 1734 | 1742 | 2049 | 2052 | 2038 | 2037 |
| 2045 | | | | 2525 | | | | 2044 | | | |
| 15. 3 | | | | 1004. 5 | | | | 7. 6 | | | |
| 0. 7% | | | | 39. 8% | | | | 0. 4% | | | |

## FIG. 32

| Unit period | T4 | | | | T5 | | | |
|---|---|---|---|---|---|---|---|---|
| Subperiod | PT1 | PT2 | PT3 | PT4 | PT1 | PT2 | PT3 | PT4 |
| AVEp | 2091 | 2030 | 2063 | 2040 | 2077 | 2047 | 2062 | 2012 |
| AVEap | 2056 | | | | 2050 | | | |
| $\sigma$ ap | 27.5 | | | | 27.8 | | | |
| CV | 1.3% | | | | 1.4% | | | |

| | T6 | | | | T7 | | | | T8 | | | |
|---|---|---|---|---|---|---|---|---|---|---|---|---|
| | PT1 | PT2 | PT3 | PT4 | PT1 | PT2 | PT3 | PT4 | PT1 | PT2 | PT3 | PT4 |
| | 2089 | 2071 | 2028 | 2061 | 2839 | 1113 | 2083 | 2047 | 2071 | 2031 | 2070 | 2035 |
| | 2062 | | | | 2021 | | | | 2052 | | | |
| | 25.5 | | | | 706.5 | | | | 21.8 | | | |
| | 1.2% | | | | 35.0% | | | | 1.1% | | | |

FIG. 33

| Unit period | T4 | | | | T5 | | | |
|---|---|---|---|---|---|---|---|---|
| Subperiod | PT1 | PT2 | PT3 | PT4 | PT1 | PT2 | PT3 | PT4 |
| AVEp | 2117 | 2076 | 2034 | 2047 | 2074 | 2055 | 2017 | 2066 |
| AVEap | 2068 | | | | 2053 | | | |
| σap | 36. 6 | | | | 25. 2 | | | |
| CV | 1. 8% | | | | 1. 2% | | | |

| | T6 | | | | T7 | | | | T8 | | | |
|---|---|---|---|---|---|---|---|---|---|---|---|---|
| | PT1 | PT2 | PT3 | PT4 | PT1 | PT2 | PT3 | PT4 | PT1 | PT2 | PT3 | PT4 |
| | 2159 | 1989 | 2086 | 2108 | 1773 | 2949 | 1116 | 1873 | 2122 | 2019 | 2110 | 2062 |
| | 2085 | | | | 1928 | | | | 2078 | | | |
| | 71. 2 | | | | 758. 8 | | | | 47. 3 | | | |
| | 3. 4% | | | | 39. 4% | | | | 2. 3% | | | |

FIG. 34

| Unit period | T4 | | | | T5 | | | |
|---|---|---|---|---|---|---|---|---|
| Subperiod | PT1 | PT2 | PT3 | PT4 | PT1 | PT2 | PT3 | PT4 |
| AVEp | 2068 | 2048 | 2033 | 2062 | 2050 | 2069 | 2040 | 2041 |
| AVEap | 2053 | | | | 2050 | | | |
| σ ap | 15. 7 | | | | 13. 2 | | | |
| CV | 0. 8% | | | | 0. 6% | | | |

| T6 | | | | T7 | | | | T8 | | | |
|---|---|---|---|---|---|---|---|---|---|---|---|
| PT1 | PT2 | PT3 | PT4 | PT1 | PT2 | PT3 | PT4 | PT1 | PT2 | PT3 | PT4 |
| 2061 | 2061 | 2022 | 2049 | 2046 | 2004 | 1930 | 693 | 2042 | 2036 | 2049 | 2062 |
| 2048 | | | | 1668 | | | | 2047 | | | |
| 18. 3 | | | | 652. 0 | | | | 11. 2 | | | |
| 0. 9% | | | | 39. 1% | | | | 0. 5% | | | |

## FIG. 35

| Unit period | T4 | | | | T5 | | | |
|---|---|---|---|---|---|---|---|---|
| Subperiod | PT1 | PT2 | PT3 | PT4 | PT1 | PT2 | PT3 | PT4 |
| AVEp | 2073 | 2043 | 2044 | 2059 | 2077 | 2026 | 2042 | 2056 |
| AVEap | 2055 | | | | 2050 | | | |
| $\sigma$ ap | 14. 1 | | | | 21. 5 | | | |
| CV | 0. 7% | | | | 1. 1% | | | |

| T6 | | | | T7 | | | | T8 | | | |
|---|---|---|---|---|---|---|---|---|---|---|---|
| PT1 | PT2 | PT3 | PT4 | PT1 | PT2 | PT3 | PT4 | PT1 | PT2 | PT3 | PT4 |
| 2089 | 2046 | 2012 | 2062 | 1705 | 753 | 636 | 3760 | 2056 | 2019 | 2029 | 2038 |
| 2052 | | | | 1713 | | | | 2035 | | | |
| 32. 1 | | | | 1445. 7 | | | | 15. 7 | | | |
| 1. 6% | | | | 84. 4% | | | | 0. 8% | | | |

# FIG. 36

| Unit period | T4 | | | | T5 | | | |
|---|---|---|---|---|---|---|---|---|
| Subperiod | PT1 | PT2 | PT3 | PT4 | PT1 | PT2 | PT3 | PT4 |
| AVEp | 2064 | 2058 | 2047 | 2038 | 2044 | 2044 | 2060 | 2041 |
| AVEap | 2052 | | | | 2047 | | | |
| σ ap | 11. 6 | | | | 8. 6 | | | |
| CV | 0. 6% | | | | 0. 4% | | | |

| T6 | | | | T7 | | | | T8 | | | |
|---|---|---|---|---|---|---|---|---|---|---|---|
| PT1 | PT2 | PT3 | PT4 | PT1 | PT2 | PT3 | PT4 | PT1 | PT2 | PT3 | PT4 |
| 2072 | 2026 | 2044 | 2061 | 4093 | 3354 | 1763 | 1747 | 2061 | 2023 | 2025 | 2036 |
| 2051 | | | | 2739 | | | | 2037 | | | |
| 20. 4 | | | | 1175. 7 | | | | 17. 5 | | | |
| 1. 0% | | | | 42. 9% | | | | 0. 9% | | | |

# FIG. 37

| Unit period | T4 | | | | T5 | | | |
|---|---|---|---|---|---|---|---|---|
| Subperiod | PT1 | PT2 | PT3 | PT4 | PT1 | PT2 | PT3 | PT4 |
| AVEp | 2094 | 2080 | 2087 | 2047 | 2190 | 2078 | 1935 | 2042 |
| AVEap | 2077 | | | | 2061 | | | |
| $\sigma$ap | 21.1 | | | | 105.3 | | | |
| CV | 1.0% | | | | 5.1% | | | |

| T6 | | | | T7 | | | | T8 | | | |
|---|---|---|---|---|---|---|---|---|---|---|---|
| PT1 | PT2 | PT3 | PT4 | PT1 | PT2 | PT3 | PT4 | PT1 | PT2 | PT3 | PT4 |
| 2102 | 2098 | 2019 | 1945 | 2608 | 1942 | 2021 | 2004 | 2183 | 1957 | 2046 | 2083 |
| 2041 | | | | 2144 | | | | 2067 | | | |
| 74.5 | | | | 311.3 | | | | 93.8 | | | |
| 3.7% | | | | 14.5% | | | | 4.5% | | | |

# FIG. 38

## INTERNATIONAL SEARCH REPORT

| | International application No. |
|---|---|
| | **PCT/JP2021/035075** |

### A. CLASSIFICATION OF SUBJECT MATTER

***G01P 5/26***(2006.01)i; ***G01F 1/66***(2022.01)i; ***G01N 21/53***(2006.01)i; ***A61B 5/0285***(2006.01)i
FI:   G01N21/53 Z; A61B5/0285 H; G01P5/26 A; G01F1/66 103

According to International Patent Classification (IPC) or to both national classification and IPC

### B. FIELDS SEARCHED

Minimum documentation searched (classification system followed by classification symbols)

G01P5/26; G01F1/66; G01N21/00-21/61; A61B5/026-5/0295

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2019/146762 A1 (KYOCERA CORP.) 01 August 2019 (2019-08-01) | 1-4, 8-12 |
| | claims 1, 12, 16, paragraphs [0011]-[0014], [0032], [0034], [0044], [0045], fig. 1-4 | |
| A | | 5-7 |
| Y | WO 2019/189630 A1 (KYOCERA CORP.) 03 October 2019 (2019-10-03) | 1-4, 8-12 |
| | paragraphs [0002], [0016], [0024], [0034], [0035], [0040], [0047]-[0049], fig. 3, 18, 19 | |
| A | | 5-7 |
| Y | Microfilm of the specification and drawings annexed to the request of Japanese Utility Model Application No. 87168/1988 (Laid-open No. 7524/1990) (KAIJO DENKI CO. LTD.) 18 January 1990 (1990-01-18), claims, page 7, line 13 to page 8, line 18, page 11, line 3 to page 12, line 3, fig. 1 | 1-4, 8-12 |
| A | | 5-7 |
| Y | JP 2003-10188 A (HITACHI MEDICAL CORP.) 14 January 2003 (2003-01-14) | 1-4, 8-12 |
| | claims 1, 4, paragraphs [0004], [0006], [0019], [0032]-[0039], fig. 3-5 | |
| A | | 5-7 |

✓ Further documents are listed in the continuation of Box C.       ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 November 2021** | **14 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

International application No.

**PCT/JP2021/035075**

### C. DOCUMENTS CONSIDERED TO BE RELEVANT

| Category* | Citation of document, with indication, where appropriate, of the relevant passages | Relevant to claim No. |
|---|---|---|
| Y | JP 57-207882 A (TOKYO KEIKI CO., LTD.) 20 December 1982 (1982-12-20) page 2, upper left column, line 16 to lower right column, line 8, fig. 1, 2 | 1-4, 8-12 |
| A | | 5-7 |
| Y | JP 2018-179809 A (DENSO CORP.) 15 November 2018 (2018-11-15) paragraphs [0099], [0102] | 3-4 |
| A | | 5-7 |
| Y | JP 2019-45488 A (NTN CORP.) 22 March 2019 (2019-03-22) paragraphs [0032], [0050], [0051], fig. 10 | 3-4 |
| A | | 5-7 |
| A | WO 2019/082688 A1 (SONY CORP.) 02 May 2019 (2019-05-02) | 1-12 |
| A | WO 2016/046905 A1 (PIONEER CORP.) 31 March 2016 (2016-03-31) | 1-12 |
| A | US 2011/0285984 A1 (CHRISTIAN, Wiiliam R.) 24 November 2011 (2011-11-24) | 1-12 |

Form PCT/ISA/210 (second sheet) (January 2015)

## INTERNATIONAL SEARCH REPORT
### Information on patent family members

| International application No. |
|---|
| **PCT/JP2021/035075** |

| Patent document cited in search report | | | Publication date (day/month/year) | Patent family member(s) | | | Publication date (day/month/year) |
|---|---|---|---|---|---|---|---|
| WO | 2019/146762 | A1 | 01 August 2019 | US | 2021/0033438 | A1 | |
| | | | | claims 1, 12, 16, paragraphs [0022]-[0024], [0045], [0047], [0057], [0058], fig. 1-4 | | | |
| | | | | EP | 3745096 | A1 | |
| | | | | CN | 111684239 | A | |
| WO | 2019/189630 | A1 | 03 October 2019 | US | 2021/0025742 | A1 | |
| | | | | paragraphs [0002], [0040], [0048], [0058], [0059], [0064], [0069]-[0071], fig. 3, 18, 19 | | | |
| | | | | EP | 3779373 | A1 | |
| | | | | CN | 111919093 | A | |
| JP | 2-7524 | U1 | 18 January 1990 | (Family: none) | | | |
| JP | 2003-10188 | A | 14 January 2003 | US | 2004/0242979 | A1 | |
| | | | | claim 1, paragraphs [0004], [0006], [0027], [0042]-[0048], fig. 3-5 | | | |
| | | | | WO | 2003/002004 | A1 | |
| | | | | EP | 1402820 | A1 | |
| | | | | CN | 1520274 | A | |
| JP | 57-207882 | A | 20 December 1982 | (Family: none) | | | |
| JP | 2018-179809 | A | 15 November 2018 | US | 2020/0003597 | A1 | |
| | | | | [0121][0125] | | | |
| | | | | WO | 2018/190059 | A1 | |
| JP | 2019-45488 | A | 22 March 2019 | WO | 2019/044955 | A1 | |
| WO | 2019/082688 | A1 | 02 May 2019 | US | 2021/0186345 | A1 | |
| | | | | JP | 2019-76641 | A | |
| WO | 2016/046905 | A1 | 31 March 2016 | (Family: none) | | | |
| US | 2011/0285984 | A1 | 24 November 2011 | US | 2013/0215410 | A1 | |
| | | | | US | 2013/0215411 | A1 | |
| | | | | US | 9709594 | B1 | |
| | | | | EP | 2388614 | A2 | |
| | | | | CA | 2741068 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020162391 A
- JP 6237998 A
- JP 2018163163 A